# EUROPEAN PATENT APPLICATION

(11) **EP 3 418 270 A1**
(43) Date of publication of application: **26.12.2018**
(21) Application number: 17382372.5
(22) Date of filing: 19.06.2017
(51) Int. Cl.: C07C 317/14, A61K 31/222, A61P 25/16

(54) **NOVEL BIPHENYLSULFOXIMINES AS ALLOSTERIC MODULATORS OF THE DOPAMINE D1 RECEPTOR**

(71) Applicant: Universidad Complutense De Madrid, 28040 Madrid (ES); Universidade de Santiago de Compostela, 15782 Santiago de Compostela (ES)
(72) Inventor: López Rodríguez, María Luz, 28040 MADRID (ES); Benhamú Salama, Bellinda, 28100 ALCOBENDAS (ES); Vázquez Villa, María Del Henar, 45280 OLÍAS DEL REY (ES); García Cárceles, Javier, 28032 MADRID (ES); Rodriguez De Fonseca, Fernando, 29730 MÁLAGA (ES); Brea Floriani, José Manuel, 15706 SANTIAGO DE COMPOSTELA (ES); Loza Garcia, María Isabel, 15706 SANTIAGO DE COMPOSTELA (ES)
(74) Representative: ZBM Patents - Zea, Barlocci & Markvardsen

(57) **Abstract**

The present invention relates to new biphenylsulfoximine compounds and, in particular, to their activity as positive allosteric modulators of the dopamine D1 receptor and to their use for the treatment of pathological states for which a stimulator of this receptor activity is indicated. The invention further relates to pharmaceutical compositions containing them and to a process for the preparation of such compounds.

## Description

### FIELD OF THE INVENTION

The present invention relates to biphenylsulfoximine compounds and, in particular, to their activity as positive allosteric modulators of the dopamine D1 receptor and to their use for the treatment of pathological states for which a stimulator of this receptor activity is indicated. The invention further relates to pharmaceutical compositions containing them, to their use in therapy and to a process for the preparation of such compounds.

### BACKGROUND ART

Dopamine is a catecholamine neurotransmitter involved in the fine tuning of the most important brain functions including memory and cognition, control of movement, neuroendocrine integration and emotional control. Multiple neurologic and psychiatric disorders are related to dopamine-related dysfunctions. They include Parkinson's disease, schizophrenia, bipolar disorders, anxiety disorders and drug addiction. In addition, in the periphery, dopamine exerts important roles in cardiovascular and renal function, as well as in the endocrine regulation of body functions. This fact prompted the development of multiple therapies designed to interact with dopamine synthesis and degradation mechanisms, uptake systems and specific dopamine receptors.

Dopamine exerts its actions through five subtypes of receptors present both in central and peripheral tissues. The five cloned dopamine receptors belong to the large family of seven transmembrane domain receptor proteins coupled to G proteins and termed usually as GPCRs. Dopamine receptors are classified in two families based on their cDNA/gene sequence and their pharmacological/biochemical characteristics. While the D1 family (comprising D1 and D5 receptors) activates the adenylate cyclase, stimulating the production of the second messenger cAMP, the D2 receptor family (constituted by the dopamine D2, D3 and D4 receptors) inhibits cAMP production. Additional transduction systems help to identify these receptors. Thus, stimulation of the D2 receptor family activates the extracellular signal-regulated kinase (ERK) whereas the stimulation of the D1 family results in the activation of p38 mitogen-activated protein kinase (p38 MAPK).

Dopamine D1 receptors are the most abundant dopamine receptors in the mammalian brain, with very high density in the basal ganglia (caudate-putamen, substantia nigra, nucleus accumbens), the frontal cortex, the olfactory bulb and the hypothalamus. Based on this neuroanatomical location, the development of specific ligands for these receptors has been addressed, in order to provide medicines that, acting through D1 receptors, might restore the dopamine dysfunctions evidenced in frequent and severe disorders, such as Parkinson's disease and schizophrenia. However, the specific agonists and antagonists developed for these neurological and psychiatric disorders failed in clinical trials due to multiple reasons including inappropriate pharmacokinetics (low oral bioavailability), low intrinsic activity despite of high-affinity binding, and the presence of side effects, mainly epilepsy and cardiovascular events. Thus, the only dopamine D1 receptor agonist clinically approved was fenoldopam, for short-term management of severe hypertension.

In the last years a renewal on the interest in the development of dopamine D1 receptor-acting drugs has arose based on: a) the availability of new compounds with high selectivity and intrinsic activity at the D1 receptors, such as dihidrexidine and derivatives, that might be effective in Parkinson's disease to substitute mixed dopamine D1/D2 receptor agonists in use, such as apomorphine and b) the preclinical demonstration of the utility of selective dopamine D1 agonists for mild cognitive impairment in animal models of neurodegenerative disorders and schizophrenia. Thus, these new drugs were promoted to clinical trials in Parkinson's disease (i.e. dihidrexidine), but again adverse peripheral effects precluded further development, in addition to the rapid tolerance observed for the antiparkinsonian effects.

However the interest in dopamine D1 receptor ligands persists. The rationale for this interest is the demonstration of the role of dopamine D1 receptors present in the spines of pyramidal cells of cortical layer III in the dorsolateral prefrontal cortex (dlPFC). These neurons generate the mental representation needed for working memory, being the basis of abstract thoughts, and the reason of cognitive impairment in several neurodegenerative and psychiatric disorders. The activation of these receptors with selective dopamine D1 receptor agonists at very low doses, or in association with the stimulation of the α₇-acetylcholine receptor, produces a clear cognitive improvement. This finding is relevant in the context of the hypodopaminergic state that exists in the dlPFC of schizophrenic and Parkinson's disease patients with cognitive impairment. Importantly, the therapeutic effect is observed at low doses, disappearing or being constrained by adverse effects at higher doses (bell shaped dose-effect curve). The alleviation of cognitive impairment in these neurological disorders is an unmet clinical need that may have important repercussions on the prognosis of the disease, improving quality of life of millions of patients worldwide.

Because of the failure of previous selective dopamine D1 receptor agonists, much interest has been placed in the development of positive allosteric modulators (PAMs) that may offer solutions for the poor bioavailability, rapid tolerance and adverse side effects. PAMs represent an alternative pharmacological option to orthosteric agonists, classical agonists that interact with the native ligand-binding site. PAMs are drugs capable of increasing the affinity and/or the efficacy of the natural ligand at the receptor binding site by acting at a different site of the receptor molecule (allosteric or secondary site). The interaction of the PAM with its binding site alters the conformation of the receptor, facilitating the activation by the orthosteric ligand. Since they lack intrinsic activity and are usually small molecules, they offer multiple advantages including better safety and tolerability profiles. This pharmacological approach has been already validated in humans at the GABAergic neurotransmission. Thus, PAMs for the GABA-A receptor (i.e. diazepam) are nowadays widely used for psychiatric and neurological conditions such as anxiety or epilepsy.

In the case of the dopamine D1 receptor, a PAM active at this receptor would enhance the endogenous action of dopamine, increasing the efficacy of the natural ligand. A similar effect may be observed with other orthosteric D1 receptor agonists, whose efficacy might be enhanced when combined with a PAM. This is very relevant for efficacy and safety since low doses of a certain D1 receptor agonist devoid of toxic effects might be efficient in the therapeutic indications they were tested for, without resulting in rapid tolerance or seizures induction.

Compounds of the present invention are PAMs of the dopamine D1 receptor. Their chemical structure is different from prior compounds reported to act as PAMs at this receptor (Lewis MA, Hunihan L, Watson J, Gentles RG, Hu S, Huang Y, et al. Discovery of D1 dopamine receptor positive allosteric modulators (PAM): characterization of pharmacology and identification of residues which regulate species selectivity. J Pharmacol Exp Ther 2015; 354:340-349; Beadle DB, Coates DA, Hao J Jr., Krushinski JH, Reinhard MR, Schaus JM, Wolfangel CD. 3,4-Dihydroisoquinolin-2(1H)-yl compounds. Patent WO2014193781A1; Soriano A, Vendrell M, Gonzalez S, Mallol J, Albericio F, Royo M, Lluís C, Canela El, Franco R, Cortes A, Casadó V. A hybrid indoloquinolizidine peptide as allosteric modulator of dopamine D1 receptors. J Pharmacol Exp Ther 2010; 332(3):876-85).

Considering their PAM action at the dopamine D1 receptors, compounds of the present invention are useful for the treatment of conditions in which the pharmacological modulation of the D1 receptor might offer a benefit: Parkinson's disease and schizophrenia, including relief of associated symptoms such as mild cognitive impairment in Parkinson's disease, as well as cognitive impairment and negative symptoms in schizophrenia and autism spectrum disorder.

Compounds of the present invention are also believed to be useful in treating mild cognitive impairment associated to other neurological conditions such as symptoms of Alzheimer's disease, senile dementia, HIV-associated dementia, Lewy body dementia, vascular dementia, or frontotemporal dementia. Compounds of the present invention might be useful in improving motor symptoms in Parkinson's disease, as well as motor symptoms in other neurodegenerative disorders affecting the basal ganglia, such as Huntington's chorea, Tourette's syndrome, restless leg syndrome (RLS), or tardive dyskinesia.

In addition, compounds of present invention are believed useful in treating affective/emotional disorders including anxiety, depression (e.g., age-related depression), major depressive disorder (MDD), treatment-resistant depression (TRD), bipolar disorder, chronic apathy, anhedonia, chronic fatigue, post-traumatic stress disorder, seasonal affective disorder, social anxiety disorder, post-partum depression, or serotonin syndrome. As well, they might serve for the treatment of attention deficit hyperactivity disorder (ADHD), drowsiness, excessive daytime sleepiness, cachexia or inattention.

Because of the prominent role of dopamine in addictive disorders, these compounds might be useful for impulsivity, compulsive gambling, mild substance abuse, drug dependence and drug abuse relapse.

Because of the role of dopamine D1 receptors in cardiovascular control, these compounds might be useful for the treatment of sexual dysfunction (e.g., erectile dysfunction or post-SSRI sexual dysfunction), migraine, post-ischemic tubular necrosis, renal failure, hyponatremia, resistant edema, hypertension, congestive heart failure, cardiogenic or septic shock, or postoperative ocular hypotonia.

Finally, based on the link between dopamine and compulsive eating, these compounds might be useful for obesity, diabetes, hyperglycemia, atherosclerosis or dyslipidemia.

### SUMMARY OF THE INVENTION

Inventors have provided a series of novel biphenylsulfoximine compounds, which show significant potentiation of the endogenous dopamine effect in the D1 receptor without parallel stimulation of other dopaminergic subtypes activity. As it is shown in the Examples, their effects in human D1 receptors endogenously expressed in neuroblastoma SK-N-MC cell line have been tested. Functional assessment at the D1 receptor (agonist activity and allosteric modulation), selectivity over dopamine receptor subtypes (D2, D3, D4 and D5 receptors), and radioligand binding studies have been evaluated. The results show that the compounds of the invention are positive allosteric modulators (PAMs) of the human D1 receptor, indicating that these compounds are useful as a treatment for disorders on which dopamine D1 receptors have been proposed as a target for therapeutics, including:
- Cognitive impairment and negative symptoms in schizophrenia, bipolar disorders and autism spectrum disorder;
- Mild cognitive impairment associated to Parkinson's disease, schizophrenia, Alzheimer's disease, senile dementia, HIV-associated dementia, Lewy body dementia, vascular dementia, or frontotemporal dementia;
- Motor symptoms in Parkinson's disease, as well as motor symptoms in other neurodegenerative disorders affecting the basal gangliam such as Huntington's chorea, Tourette's syndrome, restless leg syndrome (RLS), or tardive dyskinesia;
- Symptoms of affective/emotional disorders including anxiety, depression (e.g., age-related depression), major depressive disorder (MDD), treatment-resistant depression (TRD), bipolar disorder, chronic apathy, anhedonia, chronic fatigue, post-traumatic stress disorder, seasonal affective disorder, social anxiety disorder, post-partum depression, serotonin syndrome, attention deficit hyperactivity disorder (ADHD), drowsiness, excessive daytime sleepiness, or inattention;
- Treatment of impulsivity, compulsive gambling, mild substance abuse, drug dependence and drug abuse relapse;
- Treatment of sexual dysfunction (e.g., erectile dysfunction or post-SSRI sexual dysfunction), migraine, post-ischemic tubular necrosis, renal failure, hyponatremia, resistant edema, hypertension, congestive heart failure, cardiogenic or septic shock or postoperative ocular hypotonia;
- Treatment of obesity, diabetes, hyperglycemia, atherosclerosis or dyslipidemia.

Therefore, according to the first aspect of the present invention, it provides biphenylsulfoximines of formula (I), a pharmaceutically acceptable salt thereof or any stereoisomer either of the compounds of formula (I) or of a pharmaceutically acceptable salt thereof: 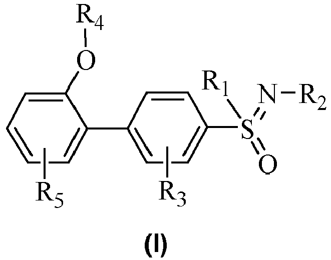 wherein:
R₁ is selected from the group consisting of (C₁-C₄)-alkyl;
R₂ is selected from the group consisting of H and (C₁₋C₄)-alkyl;
R₃ is selected from the group consisting of H, (C₁₋C₄)-alkyl and halogen;
R₄ is selected from the group consisting of CF₃, CHF₂, CH₂F, (C₁-C₄)-alkylCF₃, (C₁-C₄)-alkylCHF₂ and (C₁₋C₄)-alkylCH₂F; and
R₅ is selected from the group consisting of H, halogen, hydroxyl, (C₁₋C₄)-alkyl, methoxy and O(C₁₋C₄)-alkyl.

The present invention further provides a pharmaceutical composition comprising an effective amount of a compound of formula **(I),** a pharmaceutically acceptable salt thereof or any stereoisomer either of the compound of formula **(I)** or of a pharmaceutically acceptable salt thereof, together with appropriate amounts of one or more pharmaceutically acceptable diluents, excipients or carriers.

The present invention further provides a compound of formula **(I),** a pharmaceutically acceptable salt thereof or any stereoisomer either of the compound of formula **(I)** or of a pharmaceutically acceptable salt thereof for use in therapy.

It is also provided a compound of formula **(I),** a pharmaceutically acceptable salt thereof or any stereoisomer either of the compound of formula **(I)** or of a pharmaceutically acceptable salt thereof for use as a medicament.

The present invention further provides a compound of formula **(I),** a pharmaceutically acceptable salt thereof or any stereoisomer either of the compound of formula **(I)** or of a pharmaceutically acceptable salt thereof for use in the treatment of a disease associated with dopamine D1 receptor.

The present invention further provides a compound of formula **(I),** a pharmaceutically acceptable salt thereof or any stereoisomer either of the compound of formula **(I)** or of a pharmaceutically acceptable salt thereof for use as PAM of the human dopamine D1 receptor.

The present invention further provides a compound of formula **(I),** a pharmaceutically acceptable salt thereof or any stereoisomer either of the compound of formula **(I)** or of a pharmaceutically acceptable salt thereof for use in the treatment of a disorder selected from cognitive impairment and negative symptoms in schizophrenia, bipolar disorders and autism spectrum disorder; mild cognitive impairment associated to Parkinson's disease, schizophrenia, Alzheimer's disease, senile dementia, HIV-associated dementia, Lewy body dementia, vascular dementia, or frontotemporal dementia; motor symptoms in Parkinson's disease, as well as motor symptoms in other neurodegenerative disorders affecting the basal ganglia, such as Huntington's chorea, Tourette's syndrome, restless leg syndrome (RLS), or tardive dyskinesia; symptoms of affective/emotional disorders including anxiety, depression (e.g., age-related depression), major depressive disorder (MDD), treatment-resistant depression (TRD), bipolar disorder, chronic apathy, anhedonia, chronic fatigue, post-traumatic stress disorder, seasonal affective disorder, social anxiety disorder, post-partum depression, serotonin syndrome, attention deficit hyperactivity disorder (ADHD), drowsiness, excessive daytime sleepiness, or inattention; treatment of impulsivity, compulsive gambling, mild substance abuse, drug dependence and drug abuse relapse; treatment of sexual dysfunction (e.g., erectile dysfunction or post-SSRI sexual dysfunction), migraine, post-ischemic tubular necrosis, renal failure, hyponatremia, resistant edema, hypertension, congestive heart failure, cardiogenic or septic shock or postoperative ocular hypotonia; treatment of obesity, diabetes, hyperglycemia, atherosclerosis or dyslipidemia.

In accordance with a preferred embodiment, the disease is selected from cognitive impairment and negative symptoms in schizophrenia, bipolar disorders and autism spectrum disorder; mild cognitive impairment associated to Parkinson's disease, schizophrenia, Alzheimer's disease, senile dementia, HIV-associated dementia, Lewy body dementia, vascular dementia, or frontotemporal dementia; and motor symptoms in Parkinson's disease.

In accordance with another particular embodiment, the disease is selected from cognitive impairment and negative symptoms in schizophrenia and mild cognitive impairment associated to Parkinson's disease.

In another particularly preferred embodiment of the present invention, the disease is selected from Parkinson's disease and schizophrenia.

The present invention also relates to a method for the treatment or prevention of a disease associated with dopamine D1 receptor, comprising administering a therapeutically pharmaceutically effective amount of a compound of formula **(I),** a pharmaceutically acceptable salt thereof or any stereoisomer either of the compound of formula **(I)** or of a pharmaceutically acceptable salt thereof as defined above, together with pharmaceutically acceptable diluents, excipients or carriers, in a subject in need thereof, including a human.

The present invention further provides a method of modulating dopamine D1 receptor activity, comprising administering to a patient in need of said treatment an amount of a compound of formula **(I),** a pharmaceutically acceptable salt thereof or any stereoisomer either of the compound of formula **(I)** or of a pharmaceutically acceptable salt thereof sufficient to modulate dopamine D1 receptor activity.

The present invention also relates to a pharmaceutical composition as defined above for the manufacture of a medicament for the treatment and/or prophylaxis of a very wide range of disorders mediated by dopamine D1 receptor and associated clinical symptoms in mammals, including humans, being such disorder any of the disorders mentioned above.

Accordingly, the invention further relates to use of a compound of formula **(I),** a pharmaceutically acceptable salt thereof or any stereoisomer either of the compound of formula **(I)** or of a pharmaceutically acceptable salt thereof, as defined above, in the manufacture of a medicament for the treatment and/or prophylaxis of a disorder mediated by dopamine D1 receptor and associated clinical symptoms in a mammal, including a human. Preferably such dopamine D1 receptor mediated disorder is any of the diseases mentioned above.

This aspect may alternatively be formulated as a method of treatment and/or prophylaxis of a mammal, including a human, suffering from or being susceptible to any of the diseases mentioned above which comprises administering to said patient a therapeutically effective amount of a compound of formula **(I),** a pharmaceutically acceptable salt thereof or any stereoisomer either of the compound of formula **(I)** or of a pharmaceutically acceptable salt thereof, in combination with appropriate amounts of pharmaceutically acceptable diluents or carriers.

Compounds of the invention, including salts thereof, can be prepared using a number of synthetic routes, including the general synthetic routes described below, starting from commercially available starting materials, compounds known in the literature, or from readily prepared intermediates, by employing standard synthetic methods and procedures. Standard synthetic methods and procedures for the preparation of organic compounds and functional group transformations and manipulations are known in the art and can be found in standard textbooks.

A further aspect of the invention relates to the process for the preparation of the compounds of formula **(I)** as defined herein. The process may be performed either according to method A, method B or alternatively method C.

Method A for the preparation of the compounds of formula **(I)** comprises the following steps:
A1) reacting a sulfanyl derivative of formula **(II)** 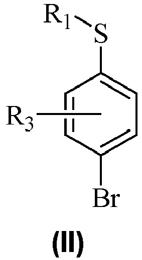 wherein R₁ and R₃ are as defined above,
   with cyanamide followed by oxidation, to obtain the corresponding intermediate *N*-cyanosulfoximine of formula **(III)** 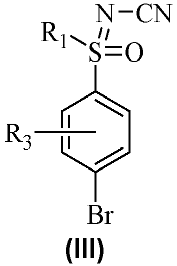 wherein R₁ and R₃ are as defined above;
A2) acidic hydrolysis of the corresponding intermediate **(III),** optionally followed by *N*-alkylation, to afford the desired intermediate sulfoximine of formula **(IV)** 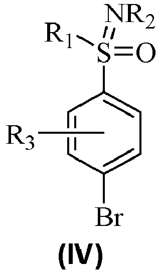 wherein R₁, R₂ and R₃ are as defined above;
   and
A3) coupling of sulfoximine of formula **(IV)** with an arylboronic acid or arylboronate, optionally followed by *O*-fluoroalkylation, resulting in final compounds of formula **(I).**

Method A for the preparation of compounds of formula **(I)** is illustrated in Scheme 1.

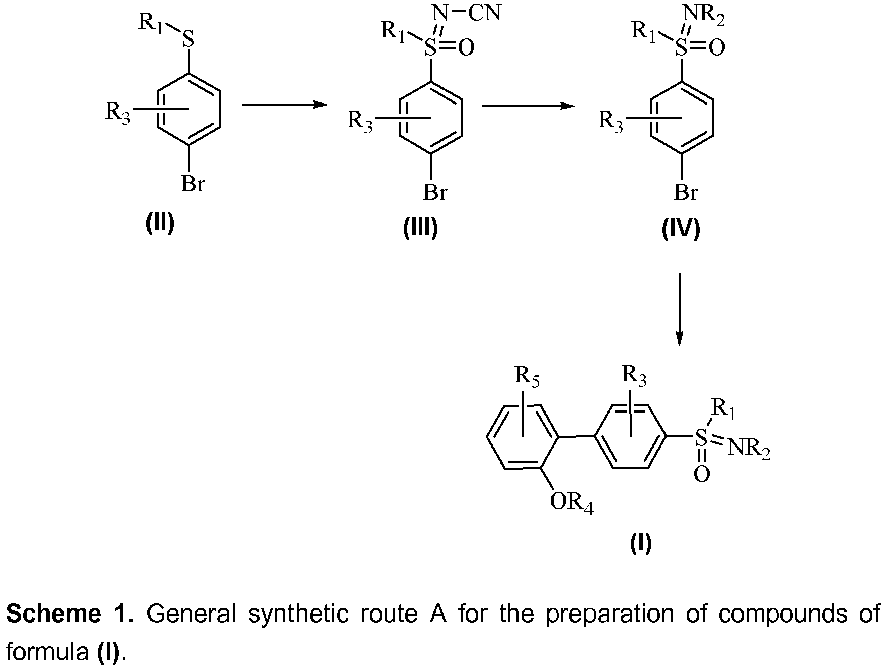

Method B for the preparation of the compounds of formula **(I)** comprises the following steps:
B1) oxidation of a sulfanyl derivative of formula **(II),** as described above, to obtain the corresponding intermediate sulfoxide of formula **(V)** 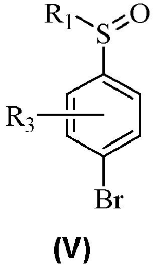 wherein R₁ and R₃ are as defined above;
B2) imination of the intermediate of formula **(V),** optionally followed by *N-*alkylation, to afford the corresponding intermediate sulfoximine of formula **(IV),** as described above 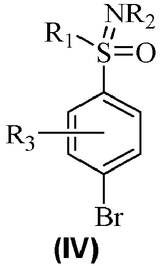 wherein R₁, R₂ and R₃ are as defined above;
   and
B3) coupling of sulfoximine of formula **(IV)** with an arylboronic acid or arylboronate, optionally followed by *O*-fluoroalkylation, resulting in final compounds of formula **(I).**

Method B for the preparation of compounds of formula **(I)** is illustrated in Scheme 2.

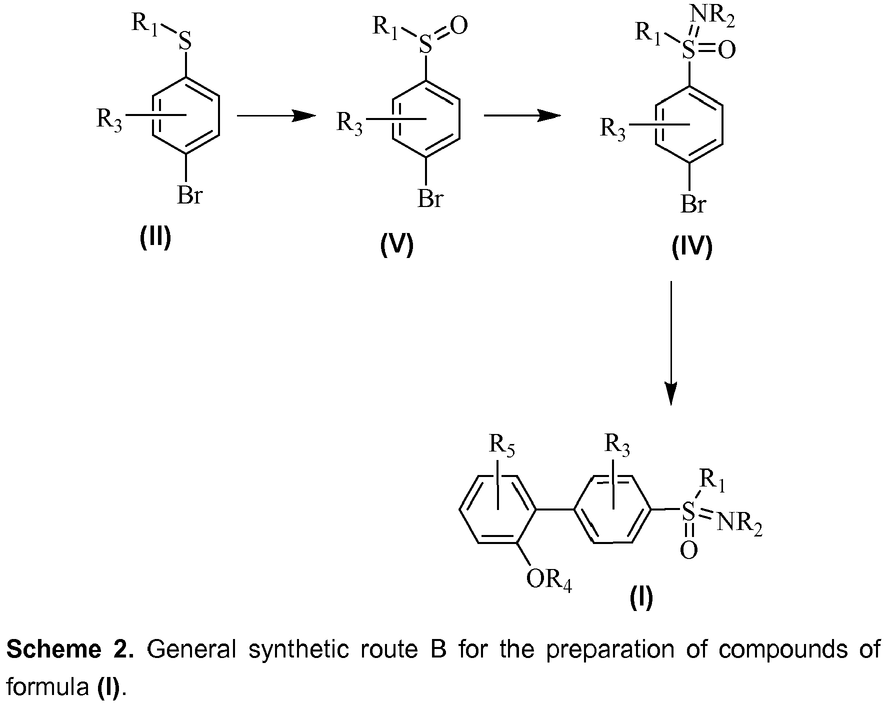

Method C for the preparation of the compounds of formula **(I)** comprises the following steps:
C1) coupling of sulfanyl derivative of formula **(II)**with an arylboronic acid or arylboronate, optionally followed by *O*-fluoroalkylation, to afford intermediate compound of formula **(VI);** 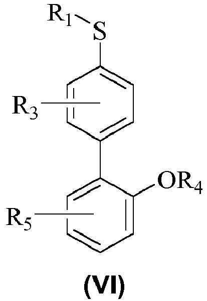 wherein R₁, R₃, R₄ and R₅ are as defined above;
C2) oxidation of resulting intermediate of formula **(VI)** to obtain the sulfoxide derivative of formula **(VII);** 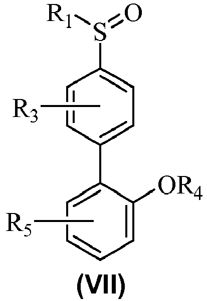 wherein R₁, R₃, R₄ and R₅ are as defined above;
   and
C3) imination of intermediate of formula **(VII),** optionally followed by *N-*alkylation, to obtain compounds of formula **(I).**

Method C for the preparation of compounds of formula **(I)** is illustrated in Scheme 3.

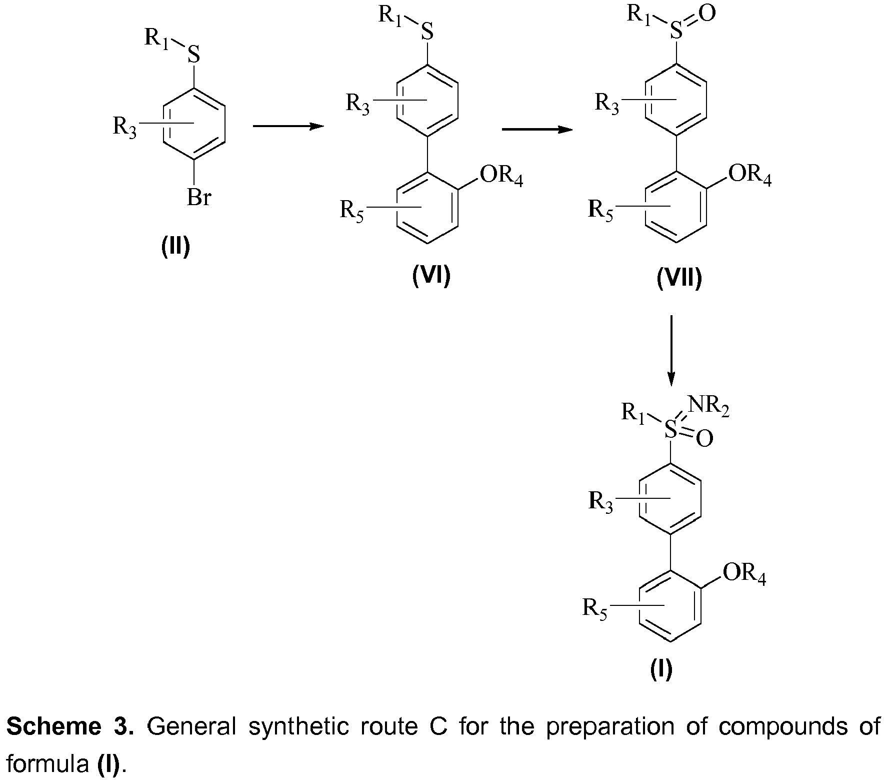

Optionally, once the compound of formula **(I)** is obtained, it can be transformed into its pharmaceutically acceptable salt by conventional processes known in the art.

Optionally, once the compound of formula **(I)** or the pharmaceutically acceptable salt thereof is obtained, it can be transformed into a prodrug thereof using methods known in the art.

This aspect of the invention may alternatively relate to the process for the preparation of enantiomerically pure compounds of formula **(I)** as defined herein.

Thus, following the synthetic route B:
B1) asymmetric oxidation of a sulfanyl derivative of formula **(II),** as described above, to obtain the corresponding enantiomerically pure sulfoxide derivative of formula (*R*)**-(V)** or (*S*)**-(V);** 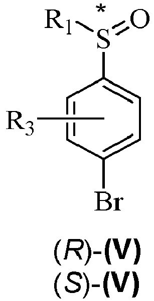 wherein R₁ and R₃ are as defined above;
B2) imination of the enantiopure intermediate of formula (*R*)**-(V)** or (*S*)-**(V)**, optionally followed by *N*-alkylation, to afford the corresponding enantiomerically pure sulfoximine intermediate of formula (*R*)**-(IV)** or (*S*)-**(IV)**, as described above; 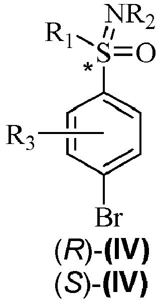 and
B3) coupling of enantiopure sulfoximine of formula (*R*)**-(IV)** or (*S*)-**(IV)** with an arylboronic acid or arylboronate, optionally followed by *O*-fluoroalkylation, resulting in enantiopure final compounds of formula (*R*)**-(I)** or (S)-**(I)**.

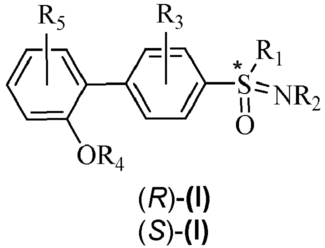

Thus, following the synthetic route C:
C1) coupling of sulfanyl derivative of formula **(II)** with an arylboronic acid or arylboronate, optionally followed by *O*-fluoroalkylation;
C2) asymmetric oxidation of resulting intermediate of formula **(VI)** to obtain enantiomerically pure sulfoxide derivative of formula (*R*)**-(VII)** or (*S*)-**(VII)** 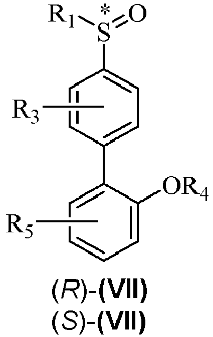 wherein R₁, R₃, R₄ and R₅ are as defined above;
   and
C3) imination of enantiopure intermediate of formula (*R*)-(**VII**) or (*S*)-(**VII**), optionally followed by *N*-alkylation, to obtain final enantiopure compound of formula (*R*)-(**I**) or (*S*)-**(I)**.

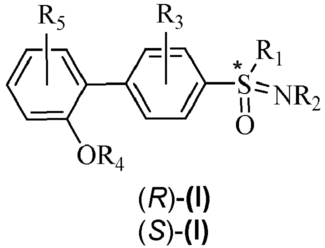

It is also part of the present invention the provision of single reaction steps of the global processes to obtain the compounds of formula **(I), (VI),** or **(VII),** and enantiomerically pure compounds of formula **(I)** or **(VII),** as well as the combination of two or more sequential steps of the global process described above, the processes being able to be carried out in sequential steps isolating the intermediates obtained, or alternatively, in one pot without isolation of any intermediate compound.

### DETAILED DESCRIPTION OF THE INVENTION

Embodiments of the present invention are outlined in the following paragraphs. It should be appreciated that certain features of the invention, which are, for clarity, described in the context of separate embodiments, can also be provided in combination with any other embodiments described herein in a single embodiment.

The compounds of the invention may have one or more asymmetric centers and may thus give rise to stereoisomers. All single optical isomers and stereoisomers, such as enantiomers, diastereoisomers and mixtures thereof, are considered within the scope of the present invention. Compounds of the present invention that contain asymmetrically substituted carbon atoms can be isolated in optically active form or racemic mixtures. Thus, any given compound referred to herein is intended to represent any one of a racemate, one or more enantiomeric forms, one or more atropoisomeric forms, and mixtures thereof.

The preparation processes described herein can be modified to give enantiopure compounds as well as mixtures of stereoisomers. It is possible to prepare specific stereoisomers or specific mixtures by various processes including the use of stereospecific reagents or by introducing chiral centers into the compounds during its preparation process. In addition, it is possible to separate stereoisomers once the compound has been prepared by standard resolution techniques known to the skilled person.

Compounds of the invention include unlabeled forms as well as isotopically labeled forms thereof. Isotopically labeled forms of the compounds are compounds that differ only in the replacement of one or more atoms by a corresponding isotopically enriched atom. Examples of isotopes that can be incorporated into compounds of the invention include for example isotopes of hydrogen, carbon, nitrogen, oxygen, fluorine, chlorine, and iodine, such as ²H, ³H, ¹¹C, ¹³C, ¹⁴C, ¹⁵N, ¹⁸O, ¹⁷O, ³⁵S, ¹⁸F, ³⁶Cl, and ¹²⁵I. Such isotopically labelled compounds are useful for example as probes in biological assays, as analytical tools, or as therapeutic agents.

The terms "compound of the invention", "compound as described herein" and the like are meant to include a compound of formula (I), including their salts, solvates, all stereoisomers, and isotopes thereof.

The present invention also includes salts of the compounds of the invention. Preferably, said salts are pharmaceutically acceptable salts.

As used herein, a "pharmaceutically acceptable salt" is intended to mean a salt that retains the biological effectiveness and properties of the parent compound (i.e. the free acid or free base, as applicable) and that is not biologically or otherwise undesirable. Pharmaceutically acceptable salts include salts formed with inorganic or organic bases, and salts formed with inorganic and organic acids.

Additionally, compounds of the present invention, or salts thereof, may exist in hydrated or unhydrated (anhydrous) form or as solvates with other solvent molecules. The term "solvate" refers to a molecular complex comprising the compound of formula **(I)** or a salt thereof, and a stoichiometric or non-stoichiometric amount of one or more solvent molecules bound by non-covalent intermolecular forces. Some compounds have a tendency to trap a fixed molar ratio of solvent molecules in the crystalline solid state, thus forming a solvate. If the solvent is water, the solvate formed is a hydrate. Non-limiting examples of solvates include hydrates and solvates with alcohols (also named alcoholates) such as ethanol (ethanolates). When compounds of the invention (or salts thereof) exist as solvates, all solvates thereof are intended to be included in the scope of the present invention, particularly pharmaceutically acceptable solvates. As used herein a "pharmaceutically acceptable solvate" is a solvate formed with a pharmaceutically acceptable solvent. Pharmaceutically acceptable solvents are well known in the art and include solvents such as water and ethanol. The compounds of the invention may be in crystalline form either as free solvation compounds or as solvates (e.g. hydrates) and it is intended that both forms are within the scope of the present invention. Methods of solvation are generally known within the art.

The reaction schemes described above are only meant as illustrative of methods to obtain the compounds of the invention. Other routes known by the ordinary skilled artisan, as well as other reactants and intermediates, can also be used to arrive at the compounds of formula **(I).**

In some of the processes described below it may be necessary or advisable to protect reactive or labile groups with conventional protecting groups. Both the nature of these protecting groups and the procedures for their introduction and removal are well known in the art. Whenever a protecting group is present, a subsequent deprotection step will be required, which can be performed under standard conditions well known in the art.

The salts of a compound of formula **(I)** can be obtained during the final isolation and purification of the compounds of the invention or can be prepared by treating a compound of formula **(I)** with a sufficient amount of the desired acid or base to give the salt in a conventional manner.

Where the processes for the preparation of the compounds of the invention give rise to mixtures of stereoisomers, individual stereoisomers of a compound of formula **(I)** can be obtained for example by resolution, starting from a compound of formula **(I)** obtained as a mixture of stereoisomers, using well known methods such as formation of diastereomeric pairs by salt formation with an optically active acid followed by fractional crystallization and regeneration of the free base, or by chiral preparative chromatography. Alternatively, it is possible to obtain optically pure or enantiomerically enriched synthetic intermediates, which can then be used as such in subsequent steps, at various stages of the synthetic procedures described above, using any known method for chiral resolution. Alternatively, it is possible to obtain optically pure or enantiomerically enriched final compounds (or synthetic intermediates) by using chiral chromatography.

Definitions of specific terms as used in the specification and claims are provided below. All other technical and scientific terms used herein and not defined below shall have the same meaning as commonly understood by one of ordinary skill in the art to which this invention pertains. In the case of conflict, the present specification, including definitions, will control.

In the case of conflict between the chemical structures and names of the compounds disclosed herein, the chemical structures will control.

"Halo" or "halogen" refers to bromo, chloro, fluoro or iodo. Preferably, halo is chloro.

As used herein, the term "hydroxyl" or "hydroxy" refers to -OH.

Combinations of substituents and variables envisioned by this invention are only those that result in the formation of stable compounds. The term "stable", as used herein, refers to compounds which possess stability sufficient to allow manufacture and which maintains the integrity of the compound for a sufficient period of time to be useful for the purposes detailed herein (e.g., therapeutic administration to a subject).

According to an embodiment of the invention, it provides biphenylsulfoximine compounds of formula **(I)** wherein
R₁ is selected from -CH₃, -CH₂CH₃, -CH₂CH₂CH₃ and -CHCH₃CH₃;
R₂ is selected from H, -CH₃, -CH₂CH₃, -CH₂CH₂CH₃ and -CHCH₃CH₃; and
R₄ is selected from CF₃, CHF₂, CH₂F, -CH₂CF₃, -CH₂CHF₂, -CH₂CH₂F, -CH₂CH₂CF₃, -CH₂CH₂CHF₂ and -CH₂CH₂CH₂F.

In accordance with another embodiment of the invention, it provides biphenylsulfoximine compounds of formula **(I)** wherein
R₃ is selected from H, -CH₃, -CH₂CH₃, F, Cl and Br; and
R₅ is selected from H, F, Cl, Br, -OH, -CH₃ and -CH₂CH₃.

Preferably, the biphenylsulfoximine compounds of formula **(I)** are those wherein
R₁ is selected from -CH₃, -CH₂CH₃, -CH₂CH₂CH₃ and -CHCH₃CH₃;
R₂ is selected from H, -CH₃, -CH₂CH₃, -CH₂CH₂CH₃ and -CHCH₃CH₃;
R₃ is selected from H, -CH₃, -CH₂CH₃, F, Cl and Br;
R₄ is selected from CF₃, CHF₂, CH₂F, -CH₂CF₃, -CH₂CHF₂, -CH₂CH₂F, -CH₂CH₂CF₃, -CH₂CH₂CHF₂ and -CH₂CH₂CH₂F; and
R₅ is selected from H, F, Cl, Br, -OH, -CH₃ and -CH₂CH₃.

In another preferred embodiment, the invention provides biphenylsulfoximine compounds of formula **(I)** wherein
R₁ is selected from -CH₃ and -CH₂CH₃;
R₂ is selected from H and -CH₃;
R₃ is selected from H and Cl;
R₄ is selected from -CF₃, -CHF₂ and -CH₂F; and
R₅ is H.

The following compounds are particularly preferred: 2-(difluoromethoxy)-4'-(*S*-methylsulfonimidoyl)biphenyl **(Ia)**; 2-(difluoromethoxy)-4'-(*N*,*S*-dimethylsulfonimidoyl)biphenyl (**Ib**)**;** 2-(difluoromethoxy)-4'-(*S*-ethylsulfonimidoyl)biphenyl **(Ic)**; 3'-chloro-2-(difluoromethoxy)-4'-(*S*-methylsulfonimidoyl)biphenyl **(Id)**; 3'-chloro-2-(difluoromethoxy)-4'-(*N,S*-dimethylsulfonimidoyl)biphenyl **(Ie)**; 3'-chloro-4'-(*S*-methylsulfonimidoyl)-2-(trifluoromethoxy)biphenyl (If); 3'-chloro-2-(trifluoromethoxy)-4'-(*N*,*S*-dimethylsulfonimidoyl)biphenyl **(Ig)**; 4'-(*N,S*-dimethylsulfonimidoyl)-2-(fluoromethoxy)biphenyl **(Ih)**; 2-(fluoromethoxy)-4'-(*S*-ethylsulfonimidoyl)biphenyl **(Ii)**; 3'-chloro-2-(fluoromethoxy)-4'-(*N*,S-dimethylsulfonimidoyl)biphenyl **(Ij)**; 2-(fluoromethoxy)-4'-(*S*-methylsulfonimidoyl)biphenyl **(Ik)**; (R)-2-(fluoromethoxy)-4'-(*S*-methylsulfonimidoyl)biphenyl **(**(*R*)-**Ik)**; (S)-2-(fluoromethoxy)-4'-(*S*-methylsulfonimidoyl)biphenyl **(**(*S*)-**Ik)**.

The final products have been structurally characterized by IR, NMR and MS techniques.

As it is shown in the Examples, compounds of the invention potently enhanced dopamine maximum effect in human cells expressing the D1 receptor, without exhibiting intrinsic agonist activity in the receptor. The compounds of the invention do not stimulate dopaminergic D2, D3, D4 and D5 receptors and do not displace the selective D1 receptor reference ligand SCH-23390 (CAS number 87075-17-0) from its orthosteric binding site.

Taken together, the results show that compounds of the invention are potent and specific allosteric potentiators of the dopamine D1 receptor in human cells.

In the field of drug design, any structural modification of a pharmacologically active compound was, in the absence of an established correlation between structural features and activity, expected a priori to disturb the pharmacological activity profile of the initial structure.

The compounds of the invention are structurally different from the compounds described in the prior art because of the novel combination of substituents of the formula, and particularly in the specific selection of the sulfoximine moiety present in their structure. These structural variations are neither disclosed nor suggested in the prior art. These structural variations result in compounds that are useful as PAMs of the dopamine D1 receptor, with a remarkable potentiation of the dopamine effect in human cell lines.

Unless otherwise stated, any description of a method of treatment includes use of the compounds to provide such treatment as is described herein, as well as use of the compounds to prepare a medicament to treat such condition.

As used herein, the term "subject" or "patient" or "individual" refers to any animals, including mammals, preferably humans.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations. Accordingly, a therapeutically effective amount of a compound may be an amount which is sufficient to treat a disease or disorder, delay the onset or progression of a disease or disorder, and/or alleviate one or more symptoms of the disease or disorder, when administered to a subject suffering from said disease or disorder. The precise effective amount for a subject will depend upon a variety of factors such as the subject's body weight, size and health, the nature and extent of the condition to be treated, and the therapeutic or combination of therapeutics selected for administration. Therapeutically effective amounts for a given situation can be determined by routine experimentation that is within the skill and judgement of the clinician.

For any compound, the therapeutically effective amount can be estimated initially either in in vitro assays, e.g. cell culture assays, or in animal models, e.g. mice, rats or dogs. The animal model may also be used to determine the appropriate concentration range and route of administration. Such information can then be used to determine useful doses and routes for administration in humans. Therapeutic efficacy and toxicity may be determined by standard procedures in cell cultures or experimental animals, e.g. ED₅₀ and LD₅₀ values can be determined and the ratio between toxic and therapeutic effects, also known as therapeutic index, may be calculated and used to determine suitable doses for use in humans.

As used herein, unless otherwise stated, the term "treating" and "treatment" in relation to a disease, disorder or condition refers to the management and care of a patient for the purpose of combating a disease, disorder or condition, such as to reverse, alleviate, inhibit the process of, or prevent the disease, disorder or condition to which such term applies, or one or more symptoms of such disease, disorder or condition, and includes the administration of a compound of the invention (or a pharmaceutically acceptable salt thereof) to prevent the onset of the symptoms or the complications, or alleviating the symptoms or complications, or eliminating the disease, condition or disorder. Preferably, treatment is curative or ameliorating.

While it is possible that a compound of the invention may be administered for use in therapy directly as such, it is typically administered in the form of a pharmaceutical composition. These compositions comprise a compound of the invention (or a pharmaceutically acceptable salt thereof) as active pharmaceutical ingredient together with one or more pharmaceutically acceptable carriers. For the purposes of the invention, a carrier is suitable for use in the pharmaceutical compositions described herein if it is compatible with the other ingredients of the composition and not deleterious to the recipient of the composition. The expression "pharmaceutically acceptable diluents, excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. A "pharmaceutically acceptable carrier" includes non-API (API refers to Active Pharmaceutical Ingredient) substances, such as disintegrators, binders, fillers, lubricants and the like, used in formulating pharmaceutical products and regarded as safe for administering to subjects (particularly humans) according to established governmental standards, including those promulgated by the United States Food and Drug Administration and the European Medical Agency. Pharmaceutically acceptable carriers are well known to those skilled in the art and are selected on the basis of the chosen type of formulation and route of administration, according to standard pharmaceutical practice.

Pharmaceutical compositions can be prepared in a manner well known in the pharmaceutical art, and can be administered by a variety of routes, for example via oral, parenteral, pulmonary or topical route. Parenteral administration includes intravenous, intra-arterial, subcutaneous, intraperitoneal or intramuscular. Parenteral administration can be in the form of a single bolus dose, or may be, for example, by a continuous perfusion pump. Pulmonary administration includes e.g. by inhalation or insufflation of powders or aerosols, including by nebulizer. Topical administration includes transdermal, epidermal, ophthalmic and to mucous membranes including intranasal, vaginal and rectal delivery.

The compositions can be formulated as to provide quick (immediate), sustained or delayed release of the active ingredient after administration to the patient by using methods known in the art.

Examples of pharmaceutically acceptable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, and methyl cellulose. The pharmaceutical compositions can additionally include further pharmaceutically acceptable excipients including: lubricating agents such as talc, magnesium stearate and mineral oil; wetting agents; emulsifying and suspending agents; preserving agents such as methyl- and propylhydroxybenzoates; sweetening agents; flavouring agents; and colouring agents.

Suitable oral dosage forms include, for examples, tablets, pills, sachets or capsules of hard or soft gelatin or any other suitable material. For example, the active compound can be incorporated into a formulation that includes pharmaceutically acceptable carriers such as binders (e.g., gelatin, cellulose, gum tragacanth), excipients (e.g., starch, lactose), lubricants (e.g., magnesium stearate, silicon dioxide), disintegrating agents (e.g., alginate, Primogel, corn starch), and sweetening or flavoring agents (e.g., glucose, sucrose, saccharin, methyl salicylate, and peppermint). They can then be compressed into tablets or enclosed in capsules using conventional techniques. The capsules and tablets can also be coated with various coatings known in the art to modify the flavors, tastes, colors, and shapes of the capsules and tablets. In addition, liquid carriers such as fatty oil can also be included in capsules. Oral formulations can also be in the form of suspensions, solutions, syrups and the like. If desired, conventional agents for modifying flavors, tastes, color and the like can be added.

Pharmaceutical compositions suitable for parenteral administration include sterile aqueous solutions or suspensions, or can be alternatively prepared in lyophilized form for extemporaneous preparation of a solution or suspension using a sterile aqueous carrier prior to use. In such formulations, diluents or pharmaceutically acceptable carriers such as sterile water and physiological saline buffer can be used. Other conventional solvents, pH buffers, stabilizers, anti-bacterial agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

Compositions for administration by inhalation or insufflation include solutions and suspensions in pharmaceutically acceptable aqueous or organic solvents, or mixtures thereof, and powders. The liquid or solid compositions may include suitable pharmaceutically acceptable excipients as described above. Such compositions may be administered by the oral or nasal respiratory route for local or systemic effect. Compositions can be nebulized by use of a suitable gas. Nebulized solutions may be breathed directly from the nebulizing device or the nebulizing device may be attached to a face mask or the breathing chamber. Solutions, suspensions and powder compositions can be administered orally or nasally from devices which deliver the formulation in an appropriate manner.

Pharmaceutical compositions for topical administration may include transdermal patches, ointments, lotions, creams, gels, drops, suppositories, sprays, liquids and powders. Topical formulations can contain one or more conventional carriers. For example, ointments can contain water and one or more hydrophobic carriers selected from liquid paraffin, polyoxyethylene alkyl ether, propylene glycol, white vaseline and the like. Carrier compositions of creams can be based on water in combination with glycerol and one or more other components such as cetylstearyl alcohol, glycerin monostearate and the like. Gels can be formulated using isopropyl alcohol and water, suitably in combination with other excipients such as glycerol, hydroxyethyl cellulose and the like.

The pharmaceutical compositions, like oral and parenteral compositions, can be formulated in unit dosage forms for ease of administration and uniformity of dosage. As used herein, "unit dosage forms" refers to physically discrete units suitable as unitary dosages for administration to subjects, each unit containing a predetermined quantity of active ingredient calculated to produce the desired therapeutic effect, in association with one or more suitable pharmaceutical carriers.

In therapeutic applications, pharmaceutical compositions are to be administered in a manner appropriate to the disease to be treated, as determined by a person skilled in the medical arts. An appropriate dose and suitable duration and frequency of administration will be determined by such factors as the condition of the patient, the type and severity of the disease, the particular form of the active ingredient and the method of administration, among others. In general, an appropriate dose and administration regimen provides the pharmaceutical composition in an amount sufficient to provide therapeutic benefit, for example an improved clinical outcome, such as more frequent complete or partial remissions, or longer disease-free and/or overall survival, or lessening of symptoms severity, or any other objectively identifiable improvement as noted by the clinician. Effective doses may generally be assessed or extrapolated using experimental models like dose-response curves derived from in vitro or animal model test systems.

The pharmaceutical compositions of the invention can be included in a container, pack or dispenser together with instructions for administration.

The compounds of the invention can also be administered in combination with another active agent as long as the other active agent does not interfere with or adversely affect the effects of the active compounds of this invention. Combination therapy includes administration of a single pharmaceutical dosage formulation which contains a compound of the invention and one or more additional active agents, as well as administration of the compound of the invention and each additional active agent in its own separate pharmaceutical dosage formulation. If administered separately, the administration can be simultaneous, sequential or separate, and the compound of the invention and the additional therapeutic agent(s) can be administered via the same administration route or using different administration routes, for example one compound can be administered orally and the other intravenously.

Typically, for combination therapy with a compound of the invention, L-DOPA, any dopaminergic agonist, MAO inhibitor, COMT inhibitor, or cholinergic agonist may be used.

### BRIEF DESCRIPTION OF THE DRAWINGS

**Figure 1****.** Dose-response curves of dopamine, in the presence of different concentrations of (A) **Ik,** (B) (*R*)-**Ik** and (C) (*S*)-**Ik,** at the dopamine D1 receptors.
**Figure 2****.** Dose-response curves of compounds (A) **Ik,** (B) (*R*)-**Ik** and (C) (*S*)-**Ik** at the dopamine D1 receptors.
**Figure 3****.** Selectivity of compounds (A) **Ik,** (B) (*R*)-**Ik** and (C) (*S*)-**Ik** over dopamine D2 receptors.
**Figure 4****.** Selectivity of compounds (A) **Ik,** (B) (*R*)-**Ik** and (C) (*S*)-**Ik** over dopamine D3 receptors.
**Figure 5****.** Selectivity of compounds (A) **Ik,** (B) (*R*)-**Ik** and (C) (*S*)-**Ik** over dopamine D4 receptors.
**Figure 6****.** Selectivity of compounds (A) **Ik,** (B) (*R*)-**Ik** and (C) (*S*)-**Ik** over dopamine D5 receptors.

Throughout the description and claims, the word "comprise" and variations of the word are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### DETAILED DESCRIPTION OF PARTICULAR EMBODIMENTS

### EXAMPLE 1. General procedure for the synthesis of intermediates of formula (II)(See Schemes 1-3).

A mixture of the corresponding benzenethiol derivative (1.0 equiv), alkyliodide (1.1 equiv) and K₂COₛ (1.2 equiv) in anhydrous acetone (1.5 mL/mmol) was stirred at rt until consumption of the starting material. Next, the reaction was poured into water and extracted with EtOAc (x3). The combined organic layers were washed with brine, dried over Na₂SO₄, filtered and concentrated under reduced pressure. The crude product was purified by column chromatography, if necessary, to obtain intermediate of formula **(II).**

4-Bromo-2-chloro-1-(methylsulfanyl)benzene **(IIa).** Following the previous general procedure, intermediate **IIa** was obtained starting from 4-bromo-2-chlorobenzenethiol (520 mg, 2.32 mmol) as an oil (575 mg, quantitative), which was used in the next step without further purification. 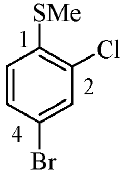 R_{f}: 0.46 (hexane/DCM 9:1). IR (ATR): v 1443 (Ar), 1118, 1028 (C-Hal). ¹H-NMR (CDCl₃): δ 2.46 (s, 3H, CH₃), 7.00 (d, *J* = 8.5, 1 H, H₆), 7.36 (dd, J = 8.5, 2.1, 1 H, H₅), 7.49 (d, *J* = 2.1, 1 H, H₃). ¹³C-NMR (CDCl₃): δ 15.4 (CH₃), 118.0 (C₄), 126.7 (C₆), 130.4, 132.0 (C₃, C₅), 132.6 (C₂), 137.3 (C₁). CAS registry number: 101084-82-6.

1-Bromo-2-chloro-4-(methylsulfanyl)benzene **(IIb)**. Following the previous general procedure, intermediate **IIb** was obtained starting from 4-bromo-3-chlorobenzenethiol (980 mg, 4.38 mmol) as an oil (586 mg, 56%). Chromatography: hexane. 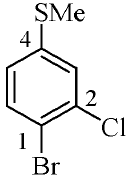 R_{f}: 0.42 (hexane/DCM 95:5). IR (ATR): v 1567 (Ar), 1100, 1014 (C-Hal). ¹H-NMR (CDCl₃): δ 2.47 (s, 3H, CH₃), 6.98 (dd, *J* = 8.4, 2.2, 1 H, H₅), 7.29 (d, *J =* 2.2, 1 H, H₃), 7.48 (d, *J =* 8.4, 1 H, H₆). ¹³C-NMR (CDCl₃): δ 15.8 (CH₃), 118.4 (C₁), 126.0 (C₅), 127.6 (C₃), 133.7 (C₆), 135.0 (C₂), 140.0 (C₄). CAS registry number: 101083-98-1.

### EXAMPLE 2. General procedure for the synthesis of intermediates of formula (III) (See Scheme 1).

*N*-bromosuccinimide (1.5 equiv) was added to a solution of the corresponding intermediate of formula **(II)** (1.0 equiv), potassium *tert*-butoxide (1.2 equiv) and cyanamide (1.3 equiv) in anhydrous methanol (5 mL/mmol) under an argon atmosphere, and the reaction mixture was stirred at rt until consumption of the starting material. Next, the solvent was evaporated under reduced pressure and the resulting residue was partitioned between DCM and water. The aqueous phase was extracted with DCM (x2) and the combined organic layers were dried over Na₂SO₄, filtered and evaporated under reduced pressure to afford the corresponding *N*-cyanosulfylimine, which was used in the next step without further purification.

To a solution of the *N*-cyanosulfylimine above synthesized in a 1:1 mixture of anhydrous acetonitrile/DCM (5 mL/mmol) under an argon atmosphere, RuCl₃ (0.01 equiv) was added and the resulting brown mixture was stirred at rt for 5 min. Then a 0.15 M solution of NalO₄ in water (1.50 equiv) was added and the reaction was stirred at rt until completion (TLC). Next, water was added and the mixture was extracted with DCM (x3). The combined organic layers were washed with sat. aqueous Na₂S₂O₃ (x2) and brine, dried over Na₂SO₄, filtered and concentrated under reduced pressure to afford intermediate of formula **(III).**

[(4-Bromophenyl)(methyl)oxido-λ6-sulfanylidene]cyanamide **(IIIa)**. Following the previous general procedure, intermediate **IIIa** was obtained starting from 1-bromo-4-(methylsulfanyl)benzene (0.69 g, 3.39 mmol) as a solid (0.73 g, 83% for 2 steps). 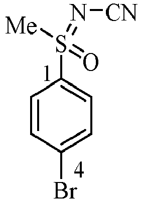 R_{f}: 0.30 (hexane/EtOAc 1:1). ¹H-NMR (CDCl₃): δ 3.34 (s, 3H, CH₃), 7.81-7.88 (m, 4H, 4CH_{Ar}). CAS registry number: 1628604-36-3.

[(4-Bromo-2-chlorophenyl)(methyl)oxido-λ⁶-sulfanylidene]cyanamide **(IIIb)**. Following the previous general procedure, intermediate **IIIb** was obtained starting from **IIa** (346 mg, 1.45 mmol) as a solid (358 mg, 84% for 2 steps). 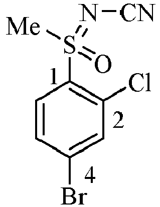 R_{f}: 0.34 (hexane/EtOAc 1:1). ¹H-NMR (CDCl₃): δ 3.53 (s, 3H, CH₃), 7.74 (dd, *J* = 8.6, 1.9, 1 H, H₅), 7.85 (d, *J* = 1.8, 1 H, H₃), 8.06 (d, *J* = 8.6, 1 H, H₆).

[(4-Bromo-3-chlorophenyl)(methyl)oxido-λ⁶-sulfanylidene]cyanamide **(IIIc)**. Following the previous general procedure, intermediate **IIIc** was obtained starting from **IIb** (248 mg, 1.04 mmol) as a solid (272 mg, 89% for 2 steps). 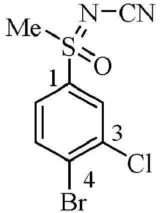 R_{f}: 0.32 (hexane/EtOAc 1:1). ¹H-NMR (CDCl₃): δ 3.36 (s, 3H, CH₃), 7.73 (dd, *J* = 8.5, 2.2, 1 H, H₆), 7.96 (d, *J* = 8.5, 1 H, H₅), 8.06 (d, *J* = 2.2, 1 H, H₂).

### EXAMPLE 3. General procedure for the synthesis of intermediates of formula (IV). Method A (See Scheme 1).

To the corresponding intermediate of formula **(III),** 50% aqueous H₂SO₄ (4.5 mL/mmol) was added and the mixture was heated at reflux for 2 h and then allowed to cool to rt. Next, neutralization at 0 °C was performed with 50% aqueous NaOH and the reaction was extracted with DCM (x3). The combined organic layers were dried over Na₂SO₄, filtered and the solvent was evaporated under reduced pressure. The crude residue was purified by column chromatography to afford the desired intermediate of formula **(IV).**

1-Bromo-4-(*S*-methylsulfonimidoyl)benzene **(IVa)**. Following the previous general procedure, intermediate **IVa** was obtained starting from **IIIa** (1.20 g, 4.63 mmol) as a white solid (0.83 g, 77%). Chromatography: hexane to hexane/EtOAc 1:1. 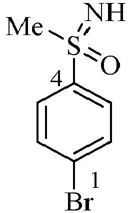 M.p.: 88-89 °C. R_{f}: 0.31 (hexane/EtOAc 2:8). IR (ATR): v 3288 (NH), 1572 (Ar), 1387, 1221 (SO). ¹H-NMR (acetone-*d6*): δ 3.06-3.07 (m, 3H, CH₃), 7.79 (d, *J* = 8.7, 2H, H₂, H₆), 7.92 (d, *J* = 8.7, 2H, H₃, H₅). ¹³C-NMR (acetone-*d6*): δ 46.5 (CH₃), 127.6 (C₁), 130.6 (C₃, C₅), 133.0 (C₂, C₆), 145.0 (C₄). MS (ESI, *m*/*z,* %): 233.7 ([M(⁷⁹Br) + H]⁺, 99), 235.7 ([M(⁸¹Br) + H]⁺, 100). CAS registry number: 101083-98-1.

1-Bromo-4-(*N,S*-dimethylsulfonimidoyl)benzene **(IVb).** A mixture of intermediate **IVa** (103 mg, 0.442 mmol), formaldehyde (37% in water, 2.0 mL) and formic acid (3.5 mL) was heated in an open flask at 100 °C for 2 days. After evaporating the volatile substances under reduced pressure, the aqueous phase was neutralized using solid NaHCO₃ and extracted with DCM (x3). The combined organic layers were dried over Na₂SO₄, filtered and concentrated under reduced pressure to obtain intermediate **IVb** as an oil (110 mg, quantitative), which was used in the next step without further purification. 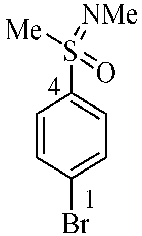 R_{f}: 0.41 (DCM/methanol 95:5). IR (ATR): v 1571 (Ar), 1242, 1151 (SO). ¹H-NMR (acetone-*d6*): δ 2.54 (s, 3H, NCH₃), 3.15 (s, 3H, SCH₃), 7.84 (m, 4H, 4CH_{Ar}). ¹³C-NMR (acetone-*d6*): δ 29.2 (NCH₃), 44.4 (SCH₃), 128.1 (C₁), 131.6 (C₃, C₅), 133.5 (C₂, C₆), 139.5 (C₄). MS (ESI, *m*/*z,* %): 248.0 ([M(⁷⁹Br) + H]⁺, 99), 250.0 ([M(⁸¹Br) + H]⁺, 100). CAS registry number: 1572928-15-4.

4-Bromo-2-chloro-1-(S-methylsulfonimidoyl)benzene (I**Vc)**. Following the previous general procedure, intermediate **IVc** was obtained starting from **IIIb** (70 mg, 0.238 mmol) as a white solid (33 mg, 51%). Chromatography: hexane to hexane/EtOAc 6:4. 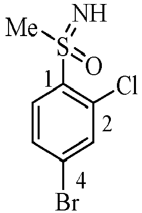 M.p.: 82-83 °C. R_{f}: 0.28 (hexane/EtOAc 1:1). IR (ATR): v 3275 (NH), 1227 (SO). ¹H-NMR (CDCl₃): δ 3.30 (s, 3H, CH₃), 7.60 (dd, *J* = 8.5, 1.9, 1 H, H₅), 7.72 (d, *J* = 1.8, 1 H, H₃), 8.05 (d, *J* = 8.5, 1 H, H₆). ¹³C-NMR (CDCl₃): δ 43.6 (CH₃), 128.2 (C₄), 130.8, 132.0 (C₃, C₅), 133.5 (C₂), 134.7 (C₆), 140.3 (C₁). MS (ESI, *m*/*z,* %): 267.8 ([M(⁷⁹Br **,** ³⁵Cl) + H]⁺, 76), 269.8 ([M(⁷⁹Br **,** ³⁷Cl) + H]⁺, 100), 271.9 ([M(⁸¹Br, ³⁷Cl) + H]⁺, 28).

4-Bromo-2-chloro-1-(*N*,*S*-dimethylsulfonimidoyl)benzene **(IVd).** A mixture of intermediate **IVc** (65 mg, 0.243 mmol), formaldehyde (37% in water, 1.1 mL) and formic acid (1.9 mL) was heated in an open flask at 100 °C for 2 days. After evaporating the volatile substances under reduced pressure, the aqueous phase was neutralized using solid NaHCO₃ and extracted with DCM (x3). The combined organic layers were dried over Na₂SO₄, filtered and concentrated under reduced pressure. The crude product was purified by column chromatography using a gradient from hexane to hexane/EtOAc 7:3, to afford intermediate I**Vd** as an oil (32 mg, 47%). 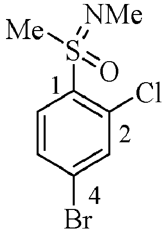 R_{f}: 0.26 (hexane/EtOAc 1:1). IR (ATR): v 1560 (Ar), 1247 (SO). ¹H-NMR (CDCl₃): δ 2.58 (s, 3H, NCH₃), 3.23 (s, 3H, SCH₃), 7.60 (dd, *J* = 8.6, 1.9, 1 H, H₅), 7.70 (d, *J* = 1.9, 1 H, H₃), 8.00 (d, *J* = 8.5, 1 H, H₆). ¹³C-NMR (CDCl₃): δ 29.7 (NCH₃), 43.0 (SCH₃), 128.2 (C₄), 131.0 (C₆), 133.5 (C₂), 134.5 (C₃), 134.6 (C₅), 135.2 (C₁). MS (ESI, *m*/*z,* %): 281.8 ([M(⁷⁹Br , ³⁵Cl) + H]⁺, 76), 283.9 ([M(⁷⁹Br , ³⁷Cl) + H]⁺, 100), 285.9 ([M(⁸¹Bᵣ, ³⁷Cl) + H]⁺, 28).

### EXAMPLE 4. General procedure for the synthesis of intermediates of formula (V) (See Scheme 2).

To a solution of the corresponding intermediate of formula **(II)** (1.00 equiv) in anhydrous DCM (14 mL/mmol) under an argon atmosphere, a solution of *m*-chloroperbenzoic acid (77%, 1.05 equiv) in anhydrous DCM (4.8 mL/mmol) was added at a rate of 8 mL/min at 0 °C, and the reaction was stirred for 30 min before warming up to rt. After 3 h, the mixture was filtered with a short pad of silica gel and washed with sat. aqueous NaHCO₃. Next, the aqueous layer was extracted with DCM (x3), and the combined organic layers were washed with water, dried over Na₂SO₄, and concentrated under reduced pressure. The residue was purified by column chromatography to afford the desired intermediate of formula **(V).**

1-Bromo-4-(ethylsulfinyl)benzene **(Va).** Following the previous general procedure, intermediate **Va** was obtained starting from **IIc** (CAS registry number: 30506-30-0) (1.08 g, 4.97 mmol) as an oil (1.06 g, 92%). Chromatography: hexane to hexane/EtOAc 7:3. 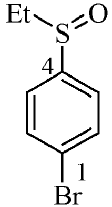 R_{f}: 0.36 (hexane/EtOAc 2:8). IR (ATR): v 1572 (Ar), 1313, 1145 (SO). ¹H-NMR (CDCl₃): δ 1.97 (t, *J* = 7.4, 3H, CH₃), 2.68-2.80 (m, 1H, 1/2CH₂), 2.85-2.96 (m, 1 H, 1/2CH₂), 7.48 (d, *J* = 8.5, 2H, H₂, H₆), 7.66 (d, *J* = 8.3, 2H, H₃, H₅). ¹³C-NMR (CDCl₃): δ 7.6 (CH₃), 50.8 (CH₂), 129.2 (C₁), 129.4 (C₃, C₅), 132.7 (C₂, C₆), 137.8 (C₄). MS (ESI, *m*/*z,* %): 233.0 ([M(⁷⁹Br) + H]⁺, 100), 235.0 ([M(⁸¹Br) + H]⁺, 100). CAS registry number: 30506-29-7.

### EXAMPLE 5. General procedure for the synthesis of intermediates of formula (IV). Method B (See Scheme 2).

A solution of the adequate intermediate of formula **(V)** (1.0 equiv) and sodium azide (1.2 equiv) in anhydrous CHCl₃ (1.0 mL/mmol) under an argon atmosphere was stirred in a pre-dried three neck round-bottom flask equipped with a reflux condenser and an addition funnel. Next, concentrated H₂SO₄ (4.6 equiv) was added dropwise at 0 °C. The resulting mixture was then slowly warmed up to 45 °C and maintained at this temperature overnight with magnetic stirring. After that time, the reaction was cooled and ice water was added. When all the salts were dissolved, the organic layer was separated and the aqueous layer was extracted with DCM first, made slightly alkaline with 20% aqueous NaOH and re-extracted with DCM (3x). The combined organic extracts were dried over Na₂SO₄, filtered and concentrated under reduced pressure. The resulting residue was purified by column chromatography to afford the desired intermediate of formula **(IV).**

1-Bromo-4-(*S*-ethylsulfonimidoyl)benzene **(IVe).** Following the previous general procedure, intermediate **IVe** was obtained starting from **Va** (0.63 g, 2.72 mmol) as an oil (0.62 g, 92%). Chromatography: DCM to DCM/methanol 96:4. 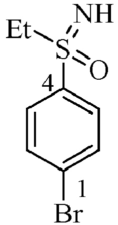 R_{f}: 0.30 (DCM/methanol 95:5). IR (ATR): v 3261 (NH), 1571 (Ar), 1213 (SO). ¹H-NMR (CDCl₃): 1δ.26 (t, *J* = 7.4, 3H, CH₃), 2.68 (br s, 1 H, NH), 3.16 (q, *J* = 7.4, 2H, CH₂), 7.69 (d, *J* = 8.7, 2H, H₂, H₆), 7.83 (d, *J* = 8.7, 2H, H₃, H₅). ¹³C-NMR (CDCl₃): δ 8.0 (CH₃), 52.0 (CH₂), 128.5 (C₁), 130.3 (C₃, C₅), 132.6 (C₂, C₆), 140.7 (C₄). MS (ESI, *m*/*z,* %): 248.0 ([M(⁷⁹Br) + H]⁺, 99), 250.0 ([M(⁸¹Br) + H]⁺, 100). CAS registry number: 1935164-83-2.

### EXAMPLE 6. General procedure for the synthesis of compounds of formula (I) Methods A and B (See Schemes 1 and 2).

A solution of intermediate of formula **(IV)** (1.00 equiv), arylboronic acid (1.05-1.20 equiv), and Na₂CO₃ (2.00 equiv) in a 2:1.5:1 mixture of toluene/water/ethanol (6.5 mL/mmol), or a solution of intermediate of formula **(IV)** (1.00 equiv), pinacol boronic ester (1.05 equiv), and K₂CO₃ (5.00 equiv) in anhydrous 1,4-dioxane (33 mL/mmol) was degassed by bubbling argon for 10 min. Pd(PPh₃)₄ (0.06 equiv) was then added and the reaction was heated under MW irradiation at 100-140 °C for 20-60 min. After this time, the mixture was extracted with EtOAc and the organic phase was dried over Na₂SO₄, filtered and evaporated under reduced pressure. The residue was purified by column chromatography or preparative TLC to afford the desired compound of formula **(I).**

### EXAMPLE 7. 2-(Difluoromethoxy)-4'-(S-methylsulfonimidoyl)biphenyl (Ia).

Following general procedure described in Example 6, compound **Ia** was obtained starting from intermediate **IVa** (42 mg, 0.181 mmol) and 2-[2-(difluoromethoxy)phenyl]-4,4,5,5-tetramethyl-1,3,2-dioxaborolane (51 mg, 0.190 mmol) as an oil (18 mg, 33%). Chromatography: DCM. 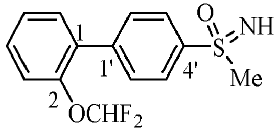 R_{f}: 0.41 (DCM/methanol 95:5). IR (ATR): v 3270 (NH), 1221 (C-O-C), 1126, 1034 (SO), 998 (C-F). ¹H-NMR (acetone-*d6*): δ 3.11 (s, 3H, CH₃), 6.98 (t, *J =* 73.9, 1 H, CHF₂), 7.35-7.42 (m, 2H, H₃, H₅), 7.52 (td, *J* = 7.4, 1.8, 1 H, H₄), 7.53 (d, *J* = 7.2, 1 H, H₆), 7.73 (d, *J* = 8.6 , 2H, H_{2'}, H_{6'}), 8.06 (d, *J* = 8.5, 2H, H_{3'}, H_{5'}) ¹³C-NMR (acetone-*d6*): δ 46.5 (CH₃), 117.6 (t, *J* = 255.8, CHF₂), 119.9 (C₃), 126.6 (C₅), 128.4 (C_{3'}, C₅), 130.8 (C₄), 130.9 (C_{2'}, C₆), 132.3 (C₆), 132.8 (C₁), 142.3 (C_{1'}), 144.5 (C₄), 149.3 (t, *J* = 2.7, C₂). ¹⁹F-NMR (acetone-*d6*): δ - 82.6. MS (ESI, *m*/*z,* %): 298.1 ([M + H]⁺, 100).

### EXAMPLE 8. 2-(Difluoromethoxy)-4'-(N,S-dimethylsulfonimidoyl)biphenyl (Ib).

A mixture of compound **Ia** (9 mg, 0.029 mmol), formaldehyde (37% in water, 0.13 mL) and formic acid (0.23 mL) was heated in an open flask at 100 °C for 2 days. After evaporating the volatile substances under reduced pressure, the aqueous phase was neutralized using solid NaHCO₃ and extracted with DCM (x3). The combined organic layers were dried over Na₂SO₄, filtered and concentrated under reduced pressure. The crude product was purified by preparative TLC in DCM/methanol 95:5, to afford compound **Ib** as an oil (5 mg, 58%). 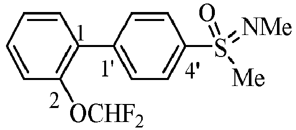 R_{f}: 0.47 (DCM/methanol 95:5). IR (ATR): v 1242 (C-O-C), 1138 (SO), 978 (C-F). ¹H-NMR (acetone-*d6*, 500 MHz): δ 2.57 (s, 3H, NCH₃), 3.10 (s, 3H, SCH₃), 6.97 (t, *J* = 73.9, 1 H, CHF₂), 7.37 (dd, *J* = 8.2, 0.6, 1 H, H₃), 7.40 (td, *J* = 7.5, 1.1, 1 H, H₅), 7.52 (ddd, *J* = 8.0, 7.9, 1.8, 1 H, H₄), 7.55 (dd, *J* = 7.7, 1.7, 1 H, H₆), 7.76 (d, *J* = 8.6, 2H, H_{2'}, H_{6'}), 7.95 (d, *J* = 8.6, 2H, H_{3',} H₅). ¹³C-NMR (acetone-*d6*, 125 MHz): δ 29.3 (NCH₃), 44.7 (SCH₃), 117.7 (t, *J* = 256.1, CHF₂), 120.0 (C₃), 126.7 (C₅), 129.4 (C_{3',}C_{5'}), 130.8 (C₄), 131.2 (C_{2'}, C_{6'}), 132.4 (C₆), 132.9 (C₁), 139.8 (C_{1'}), 144.5 (C₄), 149.4 (t, *J* = 2.9, C₂). ¹⁹F-NMR (acetone-*d6*): δ -82.6. MS (ESI, *m*/*z,* %): 312.1 ([M + H]⁺, 100).

### EXAMPLE 9. 2-(Difluoromethoxy)-4'-(S-ethylsulfonimidoyl)biphenyl (Ic).

Following general procedure described in Example 6, compound **Ic** was obtained starting from intermediate **IVe** (51 mg, 0.206 mmol) and 2-[2-(difluoromethoxy)phenyl]-4,4,5,5-tetramethyl-1,3,2-dioxaborolane (58 mg, 0.216 mmol) as an oil (14 mg, 21%). Chromatography: preparative TLC in DCM/methanol 95:5. 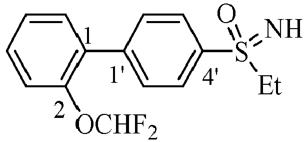 R_{f}: 0.42 (DCM/methanol 95:5). IR (ATR): v 3268 (NH), 1206 (C-O-C), 1095, 1036 (SO), 961 (C-F). ¹H-NMR (acetone-*d6*, 500 MHz): δ 1.20 (t, *J* = 7.4, 3H, CH₃), 3.18 (q, *J =* 7.4, 2H, CH₂), 6.96 (t, *J* = 73.9, 1 H, CHF₂), 7.37 (d, *J* = 8.2, 1 H, H₃), 7.40 (td, *J* = 7.6, 1.1 Hz, 1 H, H₅), 7.51 (td, *J* = 7.8, 1.7, 1 H, H₄), 7.54 (dd, *J* = 7.6, 1.6, 1 H, H₆), 7.73 (d, *J* = 8.6, 2H, H_{2'}, H_{6'}), 8.02 (d, *J* = 8.6, 2H, H_{3',} H_{5'}). ¹³C-NMR (acetone-*d6*, 125 Mz): δ 8.4 (CH₃), 52.5 (CH₂), 117.6 (t, *J =* 256.0, CHF₂), 120.0 (C₃), 126.7 (C₅), 129.3 (C_{3'}, C₅), 130.7 (C₄), 130.8 (C_{2'}, C₆), 132.3 (C₆), 132.9 (C₁), 142.4 (C_{1'}, C_{4'}), 149.3 (t, *J* = 2.8, C₂). ¹⁹F-NMR (acetone-*d6*): δ -82.5. MS (ESI, *m*/*z,* %): 312.1 ([M + H]⁺, 100).

### EXAMPLE 10. 3'-Chloro-2-(difluoromethoxy)-4'-(S-methylsulfonimidoyl)biphenyl (Id).

Following general procedure described in Example 6, compound **Id** was obtained starting from intermediate **IVc** (80 mg, 0.298 mmol) and 2-[2-(difluoromethoxy)phenyl]-4,4,5,5-tetramethyl-1,3,2-dioxaborolane (84 mg, 0.313 mmol) as an oil (28 mg, 29%). Chromatography: preparative TLC in DCM/methanol 98:2. 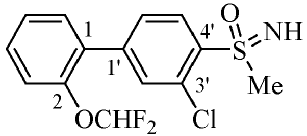 R_{f}: 0.39 (DCM/methanol 95:5). IR (ATR): v 3276 (NH), 1230 (C-O-C), 1127, 1047 (SO), 1001 (C-F). ¹H-NMR (acetone-*d6*, 500 MHz): δ 3.29 (s, 3H, CH₃), 3.82 (br s, 1 H, NH), 7.02 (t, *J* = 73.6, 1 H, CHF₂), 7.38 (br d, *J* = 7.4, 1 H, H₃), 7.40 (td, *J* = 7.6, 1.1, 1 H, H₅), 7.54 (td, *J* = 7.8, 1.7, 1 H, H₄), 7.56 (dd, *J* = 7.4, 1.6, 1 H, H₆), 7.68 (dd, *J* = 8.2, 1.7, 1 H, H_{6'}), 7.75 (d, *J* = 1.7, 1 H, H₂), 8.24 (d, *J* = *8.2,* 1 H, H_{5'}) ¹³C-NMR (acetone-*d6*, 125 MHz): δ 44.1 (CH₃), 117.6 (t, *J =* 257.9, CHF₂), 119.9 (C₃), 126.7 (C₅), 129.2 (C₆), 131.2 (C₄), 131.3 (C₅), 131.4 (C₁), 132.2 (C₆), 132.5 (C_{3'}), 133.3 (C₂), 141.9 (C₄), 143.7 (C_{1'}), 149.3 (t, *J ₌* 2.9, C₂). ¹⁹F-NMR (acetone-*d6*): δ -82.6. MS (ESI, *m*/*z,* %): 331.9 ([M(³⁵Cl) + H]⁺, 100), 333.9 ([M(³⁷Cl) + H]⁺, 38).

### EXAMPLE 11. 3'-Chloro-2-(difluoromethoxy)-4'-(N,S-dimethylsulfonimidoyl)biphenyl (Ie).

A mixture of compound **Id** (15 mg, 0.044 mmol), formaldehyde (37% in water, 0.20 mL) and formic acid (0.35 mL) was heated in an open flask at 100 °C for 2 days. After evaporating the volatile substances under reduced pressure, the aqueous phase was neutralized using solid NaHCO₃ and extracted with DCM (x3). The combined organic layers were dried over Na₂SO₄, filtered and concentrated under reduced pressure. The crude product was purified by preparative TLC in DCM/methanol 99:1, to afford compound **Ie** as an oil (10 mg, 64%). 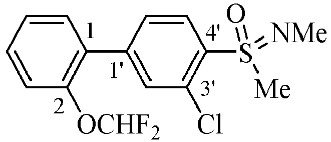 R_{f}: 0.40 (DCM/methanol 95:5). IR (ATR): v 1251 (C-O-C), 1131 (SO), 1106, 1041 (C-F). ¹H-NMR (acetone-*d6*, 500 MHz): δ 2.54 (s, 3H, NCH₃), 3.27 (s, 3H, SCH₃), 7.02 (d, *J* = 73.8, 1 H, CHF₂), 7.38 (br d, *J* = 8.5, 1 H, H₃), 7.41 (td, *J* = 7.5, 1.0, 1 H, H₅), 7.55 (td, *J* = 7.8, 1.6, 1 H, H₄), 7.58 (dd, *J* = 7.6, 1.6, 1 H, H₆), 7.72 (dd, *J* = 8.2, 1.7, 1 H, H₆), 7.78 (d, *J =* 1.6, 1 H, H_{2'}), 8.17 (d, *J =* 8.2, 1H, H_{5'}) ¹³C-NMR (acetone-*d6*, 125 MHz): δ 29.4 (NCH₃), 43.2 (SCH₃), 117.6 (t, *J =* 256.1, CHF₂), 119.9 (C₃), 126.7 (C₅), 129.5 (C₆), 131.3 (C₄), 131.4 (C₁), 132.3 (C₆), 132.7 (C_{3'}), 133.3 (C₂), 133.8 (C₅), 136.7 (C₄), 144.0 (C_{1'}), 149.3 (t, *J* = 2.8, C₂). ¹⁹F-NMR (acetone-*d6*): δ -82.7. MS (ESI, *m*/*z,* %): 346.0 ([M(³⁵Cl) + H]⁺, 100), 348.0 ([M(³⁷Cl) + H]⁺, 38).

### EXAMPLE 12. 3'-Chloro-4'-(S-methylsulfonimidoyl)-2-(trifluoromethoxy)biphenyl (If).

Following general procedure described in Example 6, compound **If** was obtained starting from intermediate **IVc** (59 mg, 0.220 mmol) and [(2-trifluoromethoxy)phenyl]boronic acid (50 mg, 0.242 mmol) as an oil (45 mg, 59%). Chromatography: preparative TLC in DCM/methanol 96:4. 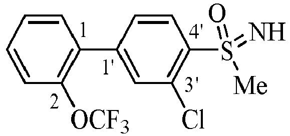 R_{f}: 0.35 (DCM/methanol 95:5). IR (ATR): v 3278 (NH), 1247, 1222 (C-O-C), 1170 (SO). ¹H-NMR (acetone-*d6*, 500 MHz): δ 3.29 (s, 3H, CH₃), 3.85 (br s, 1 H, NH), 7.52-7.54 (m, 1 H, H₃), 7.56 (td, *J* = 7.7, 1.3, 1 H, H₅), 7.60-7.65 (m, 2H, H₄, H₆), 7.68 (dd, *J* = 8.2, 1.8, 1 H, H_{6'}), 7.74 (d, *J* = 1.8, 1 H, H₂), 8.27 (d, *J* = 8.2, 1 H, H_{5'}) ¹³C-NMR (acetone-*d6*, 125 MHz): δ 44.1 (CH₃), 121.3 (q, *J* = 256.5, CF₃), 122.5 (br q, *J* = 1.5, C₃), 128.9 (C₅), 129.1 (C₆), 131.4 (C₄), 131.5 (C₅), 132.5 (C₆), 132.8 (C₁), 133.0 (C₂), 133.2 (C₃), 142.3 (C₄), 143.1 (C_{1'}), 146.7 (br q, *J* = 1.5, C₂). ¹⁹F-NMR (acetone-*d6*): δ -58.2. MS (ESI, *m*/*z,* %): 349.9 ([M(³⁵Cl) + H]⁺, 100), 351.9 ([M(³⁷Cl) + H]⁺, 38).

### EXAMPLE 13. 3'-Chloro-2-(trifluoromethoxy)-4'-(N,S-dimethylsulfonimidoyl)biphenyl (Ig).

A mixture of compound **If** (14 mg, 0.039 mmol), formaldehyde (37% in water, 0.18 mL) and formic acid (0.31 mL) was heated in an open flask at 100 °C for 2 days. After evaporating the volatile substances under reduced pressure, the aqueous phase was neutralized using solid NaHCO₃ and extracted with DCM (x3). The combined organic layers were dried over Na₂SO₄, filtered and concentrated under reduced pressure. The crude product was purified by glass column chromatography using a gradient from DCM to DCM/methanol 99:1, to afford compound **Ig** as an oil (9 mg, 61%). 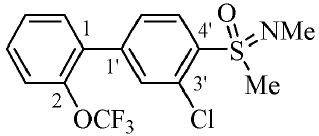 R_{f}: 0.30 (DCM/methanol 95:5). IR (ATR): v 1363 (SO), 1253, 1218 (C-O-C), 1176, 1147 (SO). ¹H-NMR (acetone-*d6*, 500 MHz): δ 2.54 (s, 3H, NCH₃), 3.28 (s, 3H, SCH₃), 7.53 (app dt, *J* = 8.1, 1.4, 1 H, H₃), 7.56 (td, *J* = 7.5, 1.2, 1 H, H₅), 7.61-7.66 (m, 2H, H₄, H₆), 7.72 (dd, *J* = 8.1, 1.8, 1 H, H_{6'}), 7.77 (d, *J* = 1.7, 1 H, H_{2'}), 8.20 (d, *J* = 8.1, 1 H, H_{5'}) ¹³C-NMR (acetone-*d6*, 125 MHz): δ 29.4 (NCH₃), 43.2 (SCH₃), 121.3 (q, *J* = 255.2, CF₃), 122.5 (br q, *J* = 1.1, C₃), 128.9 (C₅), 129.3 (C₆), 131.5 (C₄), 132.5 (C₆), 133.0 (C₁), 133.1 (C₂), 133.2 (C_{3'}), 134.0 (C₅), 137.1 (C₄), 143.4 (C_{1'}), 146.7 (br q, *J* = 1.6, C₂). ¹⁹F-NMR (acetone-*d6*): δ -58.3. MS (ESI, *m*/*z,* %): 364.0 ([M(³⁵Cl) + H]⁺, 100), 366.0 ([M(³⁷Cl) + H]⁺, 38).

### EXAMPLE 14. 4'-(N,S-Dimethylsulfonimidoyl)-2-(fluoromethoxy)biphenyl (Ih).

Following general procedure described in Example 6, 4'-(*N,S-*methylsulfonimidoyl)-1,1'-biphenyl-2-ol was obtained starting from intermediate **IVb** (108 mg, 0.434 mmol) and (2-hydroxyphenyl)boronic acid (68 mg, 0.477 mmol) as an off-white solid (51 mg, 45%). Chromatography: hexane to hexane/EtOAc 3:7. 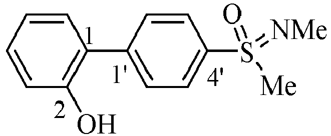 M.p.: 206-207 °C. R_{f}: 0.22 (hexane/EtOAc 1:1). IR (ATR): v 3456 (OH), 1596 (Ar), 1274, 1234 (SO). ¹H-NMR (methanol-*d4*): δ 2.63 (s, 3H, NCH₃), 3.18 (s, 3H, SCH₃), 6.91-6.96 (m, 2H, H₃, H₅), 7.22 (td, *J* = 7.9, 1.6, 1 H, H₄), 7.33 (dd, *J* = 7.3, 1.2, 1 H, H₆), 7.84-7.91 (m, 4H, H_{2'}, H_{3',} H₅, H_{6'}) ¹³C-NMR (methanol-*d4*): δ 29.6 (NCH₃), 44.6 (SCH₃), 117.1 (C₃), 121.1 (C₅), 127.9 (C₁), 129.5 (C_{3'}, C₅), 130.8 (C₄), 131.5 (C₆), 131.6 (C_{2'}, C₆), 136.6 (C₁), 146.0 (C₄), 155.8 (C₂). MS (ESI, *m*/*z,* %): 262.0 ([M + H]⁺, 100).

To a mixture of above synthesized 4'-(*N*,S-methylsulfonimidoyl)-1,1'-biphenyl-2-ol (14 mg, 0.054 mmol) and Cs₂CO₃ (28 mg, 0.086 mmol) in anhydrous DMF (0.38 mL) at -78 °C under an argon atmosphere, a solution of chlorofluoromethane (2.0 M in DMF, 108 µL, 0.216 mmol) was added dropwise. The reaction was stirred and allowed to warm to rt overnight. Afterward, the mixture was diluted with water and the aqueous layer was extracted with Et₂O (x3). The combined organic layers were dried over Na₂SO₄, filtered and concentrated under reduced pressure. The residue was purified by column chromatography in DCM to afford compound **Ih** as an oil (15 mg, 95%). 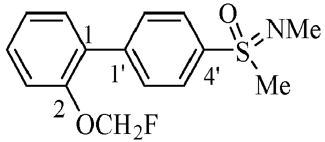 R_{f}: 0.45 (DCM/methanol 95:5). IR (ATR): v 1241, 1222 (C-O-C), 1151 (SO), 1108, 977 (C-F). ¹H-NMR (acetone-*d6*, 500 MHz): δ 2.57 (s, 3H, NCH₃), 3.08 (s, 3H, SCH₃), 5.85 (d, *J* = 54.3, 2H, CH₂F), 7.26 (td, *J* = 7.8, 1.2, 1 H, H₅), 7.36 (dd, *J* = 7.6, 1.1, 1 H, H₃), 7.46 (d, *J* = 7.5, 1 H, H₆), 7.48 (app td, *J* = 7.3, 1.2, 1 H, H₄), 7.77 (d, *J =* 8.6, 2H, H_{2'}, H_{6'}), 7.92 (d, *J =* 8.5, 2H, H_{3',} H_{5'}). ¹³C-NMR (acetone-*d6*, 125 MHz): δ 29.3 (NCH₃), 44.8 (SCH₃), 101.8 (d, *J =* 215.7, CH₂F), 116.4 (d, *J* = 1.2, C₃), 124.7 (C₅), 129.3 (C_{3',} C₅), 130.8 (C₄), 131.1 (d, *J* = 1.5, C₁), 131.2 (C_{2'}, C₆), 131.9 (C₆), 139.4 (C_{1'}), 143.2 (C₄), 154.5 (d, *J* = 3.0, C₂). ¹⁹F-NMR (acetone-*d6*): δ -151.0. MS (ESI, *m*/*z,* %): 294.1 ([M + H]⁺, 100).

### EXAMPLE 15. 2-(Fluoromethoxy)-4'-(S-ethylsulfonimidoyl)biphenyl (Ii).

Following general procedure described in Example 6, 4'-(S-ethylsulfonimidoyl)-1,1'-biphenyl-2-ol was obtained starting from intermediate **IVe** (130 mg, 0.525 mmol) and (2-hydroxyphenyl)boronic acid (87 mg, 0.630 mmol) as a solid (123 mg, 90%). Chromatography: hexane to hexane/EtOAc 1:1. 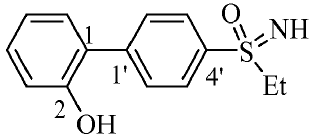 M.p.: 141-143 °C. R_{f}: 0.22 (hexane/EtOAc 2:8). IR (ATR): *v* 3283 (OH, NH), 1596, 1452 (Ar), 1277 (SO), 1207 (C-O), 1099 (SO). ¹H-NMR (acetone-*d6*): δ 1.19 (t, *J =* 7.1, 3H, CH₃), 3.16 (q, *J* = 7.4, 2H, CH₂), 3.15 (br s, 1H, NH), 6.97 (td, *J* = 7.6, 1.1, 1H, H₅), 7.04 (dd, *J* = 8.1, 0.9, 1H, H₃), 7.25 (ddd, *J* = 7.4, 7.3, 1.8, 1 H, H₄), 7.37 (dd, *J* = 7.6, 1.6, 1H, H₆), 7.82 (d, *J* = 8.5, 2H, H_{2'}, H_{6'}), 7.96 (d, *J* = 8.5, 2H, H_{3'}, H_{5'}) ¹³C-NMR (acetone-*d6*): δ 8.4 (CH₃), 52.5 (CH₂), 117.1 (C₃), 121.0 (C₅), 127.6 (C₁), 129.0 (C₃, C_{5'}), 130.4 (C₄), 130.5 (C_{2'}, C_{6'}), 131.5 (C₆), 141.1 (C_{1'}), 144.2 (C₄), 155.2 (C₂). MS (ESI, *m*/*z,* %): 260.0 ([M - H]⁻, 100).

To a mixture of above synthesized 4'-(S-ethylsulfonimidoyl)-1,1'-biphenyl-2-ol (36 mg, 0.140 mmol) and Cs₂CO₃ (73 mg, 0.224 mmol) in anhydrous DMF (1.0 mL) at -78 °C under an argon atmosphere, a solution of chlorofluoromethane (2.0 M in DMF, 280 µL, 0.560 mmol) was added dropwise. The reaction was stirred and allowed to warm to rt overnight. Afterward, the mixture was diluted with water and the aqueous layer was extracted with Et₂O (x3). The combined organic layers were dried over Na₂SO₄, filtered and concentrated under reduced pressure. The residue was purified by column chromatography in DCM to afford compound **li** as a solid (34 mg, 84%). 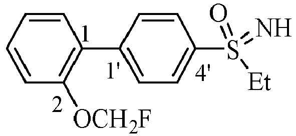 M.p.: 92-93 °C. R_{f}: 0.46 (DCM/methanol 95:5). IR (ATR): *v* 3322 (NH), 1215 (C-O-C), 1128, 1084 (SO), 973 (C-F). ¹H-NMR (acetone-*d6*): δ 1.20 (t, *J =* 7.4, 3H, CH₃), 3.17 (q, *J* = 7.4, 2H, CH₂), 5.84 (d, *J* = 54.3, 2H, CH₂F), 7.26 (td, *J* = 7.9, 1.1, 1H, H₅), 7.35 (d, *J* = 8.0, 1H, H₃), 7.46 (d, *J* = 7.7, 1H, H₆), 7.47 (td, *J* = 7.3, 1.7, 1H, H₄), 7.74 (d, *J* = 8.5, 2H, H2', H₆'), 7.99 (d, *J* = 8.5, 2H, H₃' , H₅') ¹³C-NMR (acetone-*d6*): δ 8.4 (CH₃), 52.4 (CH₂), 101.8 (d, *J =* 215.5, CH₂F), 116.4 (d, *J* = 1.1, C₃), 124.6 (C₅), 129.1 (C_{3'}, C_{5'}), 130.7 (C₄), 130.8 (C_{2'}, C_{6'}), 131.1 (d, *J* = 1.3, C₁), 131.9 (C₆), 141.9 (C_{1'}), 143.1 (C₄), 154.5 (d, *J* = 3.0, C₂). ¹⁹F-NMR (acetone-*d6*): δ -151.0. MS (ESI, *m*/*z,* %): 294.1 ([M+H]⁺, 100).

### EXAMPLE 16. 3'-Chloro-2-(fluoromethoxy)-4'-(N,S-dimethylsulfonimidoyl)biphenyl (Ij).

Following general procedure described in Example 6, 3'-chloro-4'-(N,S-dimethylsulfonimidoyl)biphenyl-2-ol was obtained starting from intermediate **IVd** (31 mg, 0.110 mmol) and (2-hydroxyphenyl)boronic acid (17 mg, 0.121 mmol) as a white solid (21 mg, 65%). Chromatography: hexane to hexane/EtOAc 8:2. 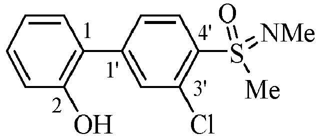 1455 (Ar), 1372 (SO), 1242, 1156 (C-O). ¹H-NMR (acetone-*d6*): δ 2.54 (s, 3H, NCH₃), 3.25 (s, 3H, SCH₃), 6.98 (td, *J* = 7.5, 1.1, 1H, H₅), 7.07 (dd, *J* = 8.1, 1.0, 1H, H₃), 7.27 (ddd, *J* = 8.1, 7.3, 1.6, 1H, H₄), 7.42 (dd, *J* = 7.7, 1.6, 1H, H₆), 7.80 (dd, *J* = 8.2, 1.7, 1H, H₆), 7.90 (d, *J =* 1.7, 1H, H_{2'}), 8.12 (d, *J =* 8.2, 1H, H₅') ¹³C-NMR (acetone-*d6*): δ 29.7 (NCH₃), 43.2 (SCH₃), 117.3 (C₃), 121.1 (C₅), 126.0 (C₁), 129.1 (C₆), 131.0 (C₄), 131.4 (C₆), 132.3 (C_{3'}), 133.1 (C_{2'}), 133.7 (C₅), 135.3 (C₄), 145.8 (C_{1'}), 155.3 (C₂). MS (ESI, *m*/*z,* %): 296.0 ([M(³⁵Cl) + H]⁺, 100), 298.0 ([M(³⁷Cl) + H]⁺, 38).

To a mixture of above synthesized 3'-chloro-4'-(N,S-dimethylsulfonimidoyl)biphenyl-2-ol (13 mg, 0.044 mmol) and Cs₂CO₃ (23 mg, 0.070 mmol) in anhydrous DMF (0.3 mL) at -78 °C under an argon atmosphere, a solution of chlorofluoromethane (2.0 M in DMF, 88 µL, 0.176 mmol) was added dropwise. The reaction was stirred and allowed to warm to rt overnight. Afterward, the mixture was diluted with water and the aqueous layer was extracted with Et₂O (x3). The combined organic layers were dried over Na₂SO₄, filtered and concentrated under reduced pressure. The residue was purified by preparative TLC in DCM/methanol 95:5 to afford compound **Ij** as an oil (9 mg, 60%). 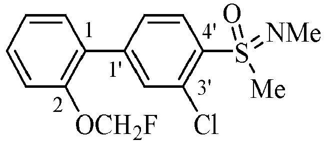 R_{f}: 0.31 (DCM/methanol 95:5). IR (ATR): *v* 1374 (SO), 1246, 1218 (C-O-C), 1149 (SO). ¹H-NMR (acetone-*d6*, 500 MHz): δ 2.54 (s, 3H, NCH₃), 3.26 (s, 3H, SCH₃), 5.89 (d, *J =* 54.2, 2H, CH₂F), 7.27 (td, *J* = 7.6, 1.0, 1H, H₅), 7.37 (d, *J =* 8.8, 1H, H₃), 7.49-7.52 (m, 2H, H₄, H₆), 7.72 (dd, *J* = 8.2, 1.7, 1H, H₆'), 7.79 (d, *J* = 1.7, 1H, H_{2'}), 8.14 (d, *J =* 8.2, 1H, H_{5'}). ¹³C-NMR (acetone-*d6*, 125 MHz): δ 29.7 (NCH₃), 43.2 (SCH₃), 101.8 (d, *J =* 217.2, CH₂F), 116.3 (d, *J =* 1.2, C₃), 124.7 (C₅), 129.5 (C₆), 129.6 (C₁), 131.3 (C₄), 131.9 (C₆), 132.5 (C₃), 133.3 (C₂), 133.7 (C₅), 136.3 (C₄), 144.8 (C_{1'}), 154.5 (d, *J =* 2.9, C₂). ¹⁹F-NMR (acetone-*d6*): δ -151.3. MS (ESI, *m*/*z,* %): 328.1 ([M(³⁵Cl) + H]⁺, 100), 330.1 ([M(³⁷Cl) + H]⁺, 41).

### EXAMPLE 17. 2-(Fluoromethoxy)-4'-(S-methylsulfonimidoyl)biphenyl (Ik).

Following general procedure described in Example 6, 4'-(S-methylsulfonimidoyl)-1,1'-biphenyl-2-ol was obtained starting from intermediate **IVa** (40 mg, 0.173 mmol) and (2-hydroxyphenyl)boronic acid (27 mg, 0.190 mmol) as a solid (24 mg, 56%). Chromatography: hexane to hexane/EtOAc 1:1. 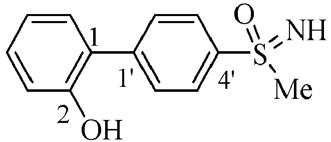 M.p.: 196-197 °C. R_{f}: 0.34 (hexane/EtOAc 2:8). IR (ATR): *v* 3279 (OH, NH), 1217 (C-O), 1100 (SO). ¹H-NMR (acetone-*d6*, 500 MHz): δ 3.09 (s, 3H, CH₃), 6.97 (td, *J* = 7.4, 1.2, 1H, H₅), 7.04 (dd, *J* = 8.1, 0.6 Hz, 1H, H₃), 7.25 (td, *J =* 8.2, 1.7, 1H, H₄), 7.37 (dd, *J* = 7.6, 1.7, 1H, H₆), 7.81 (d, *J* = 8.6, 2H, H2', H₆'), 8.01 (d, *J* = 8.4, 2H, H₃' , H_{5'}), 8.65 (s, 1H, OH). ¹³C-NMR (acetone-*d6*, 125 MHz): δ 46.7 (CH₃), 117.2 (C₃), 121.1 (C₅), 127.8 (C₁), 128.2 (C_{3'}, C_{5'}), 130.4 (C₄), 130.6 (C_{2'}, C_{6'}), 131.5 (C₆), 143.5 (C_{1'}), 144.1 (C₄), 155.2 (C₂). MS (ESI, *m*/*z,* %): 248.1 ([M + H]⁺, 100).

To a mixture of above synthesized 4'-(S-methylsulfonimidoyl)-1,1'-biphenyl-2-ol (24 mg, 0.097 mmol) and Cs₂CO₃ (51 mg, 0.155 mmol) in anhydrous DMF (0.68 mL) at -78 °C under an argon atmosphere, a solution of chlorofluoromethane (2.0 M in DMF, 194 µL, 0.388 mmol) was added dropwise. The reaction was stirred and allowed to warm to rt overnight. Afterward, the mixture was diluted with water and the aqueous layer was extracted with Et₂O (x3). The combined organic layers were dried over Na₂SO₄, filtered and concentrated under reduced pressure. The residue was purified by preparative TLC in toluene/methanol 95:5 to afford compound **Ik** as a solid (23 mg, 78%). 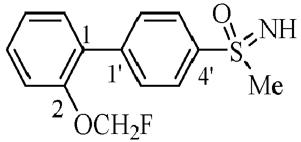 M.p.: 144-145 °C. R_{f}: 0.40 (toluene/methanol 9:1). IR (ATR): *v* 3341 (NH), 1220 (C-O-C), 1129, 1083 (SO), 994, 972 (C-F). ¹H-NMR (acetone-*d6*, 500 MHz): δ 3.10 (s, 3H, CH₃), 5.84 (d, *J* = 54.4, 2H, CH₂F), 7.26 (td, *J* = 7.5, 1.0, 1H, H₅), 7.35 (d, *J* = 8.3, 1H, H₃), 7.44-7.49 (m, 2H, H₄, H₆), 7.73 (d, *J* = 8.5, 2H, H2', H₆'), 8.04 (d, *J* = 8.5, 2H, H_{3'}, H_{5'}). ¹³C-NMR (acetone-*d6*): δ 46.6 (CH₃), 101.8 (d, *J* = 216.8, CH₂F), 116.4 (d, *J* = 1.1, C₃), 124.7 (C₅), 128.3 (C_{3'}, C_{5'}), 130.8 (C₄), 130.9 (C_{2'}, C_{6'}), 131.2 (d, *J =* 1.1, C₁), 131.9 (C₆), 143.1 (C_{1'}), 144.2 (C₄), 154.5 (d, *J* = 3.0, C₂). ¹⁹F-NMR (acetone-*d6*): δ -151.0. MS (ESI, *m*/*z,* %): 279.8 ([M + H]⁺, 100).

### EXAMPLE 18. General procedure for the synthesis of intermediates of formula (VI) (See Scheme 3).

Following general procedure described in Example 6, intermediate of formula **(VI)** was obtained starting from intermediate of formula **(II).**

2-(Fluoromethoxy)-4'-(methylsulfanyl)biphenyl **(Via).** Following the previous general procedure, 4'-(methylsulfanyl)biphenyl-2-ol was obtained starting from **IId** (CAS registry number: 104-95-0) (691 mg, 3.30 mmol) and (2-hydroxyphenyl)boronic acid (516 mg, 3.63 mmol) as an off-white solid (650 mg, 91%). Chromatography: hexane to hexane/EtOAc 9:1. 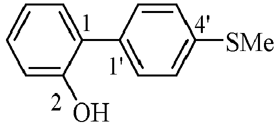 M.p.: 78-79 °C. R_{f}: 0.44 (hexane/EtOAc 6:4). IR (ATR): *v* 3530, 3425 (OH), 1480, 1447 (Ar), 1180 (C-O). ¹H-NMR (CDCl₃): δ 2.54 (s, 3H, CH₃), 5.13 (br s, 1H, OH), 6.97-7.03 (m, 2H, H₃, H₅), 7.22-7.30 (m, 2H, H₄, H₆), 7.36-7.43 (m, 4H, H2', H_{3'}, H_{5'}, H_{6'}). ¹³C-NMR (CDCl₃): δ 15.8 (CH₃), 116.0 (C₃), 121.1 (C₅), 127.2 (C_{3'}, C_{5'}), 127.7 (C₁), 129.2 (C₄), 129.6 (C_{2'}, C_{6'}), 130.3 (C₆), 133.8 (C_{1'}), 138.6 (C_{4'}), 152.6 (C₂). MS (ESI, *m*/*z,* %): 214.8 ([M - H]⁻, 100).

To a mixture of above synthesized 4'-(methylsulfanyl)biphenyl-2-ol (623 mg, 2.88 mmol) and Cs₂CO₃ (1.51 g, 4.58 mmol) in anhydrous DMF (20 mL) at -78 °C under an argon atmosphere, a solution of chlorofluoromethane (2.0 M in DMF, 5.8 mL, 11.50 mmol) was added dropwise. The reaction was stirred and allowed to warm to rt overnight. Afterward, the mixture was diluted with water and the aqueous layer was extracted with Et₂O (x3). The combined organic layers were dried over Na₂SO₄, filtered and concentrated under reduced pressure. The residue was purified by column chromatography in hexane to afford intermediate Via as an oil (600 mg, 84%). 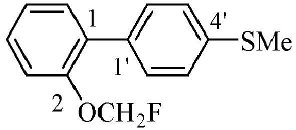 R_{f}: 0.35 (hexane/DCM 9:1). IR (ATR): *v* 1480 (Ar), 1215 (C-O-C), 971 (C-F). ¹H-NMR (CDCl₃): δ 2.54 (s, 3H, CH₃), 5.62 (d, *J* = 54.6, 2H, CH₂F), 7.17-7.26 (m, 2H, H₃, H₅), 7.32 (d, *J* = 8.5, 2H, H₃' , H_{5'}), 7.34-7.38 (m, 2H, H₄, H₆), 7.46 (d, *J =* 8.6, 2H, H2', H_{6'}). ¹³C-NMR (CDCl₃): δ 15.9 (CH₃), 101.1 (d, *J =* 219.0, CH₂F), 116.2 (d, *J* = 1.2, C₃), 124.0 (C₅), 126.3 (C_{3'}, C_{5'}), 128.9 (C₄), 130.0 (C_{2'}, C_{6'}), 131.1 (C₆), 131.6 (d, *J* = 1.3, C₁), 134.6 (C_{1'}), 137.7 (C_{4'}), 153.7 (d, *J* = 3.1, C₂). ¹⁹F-NMR (CDCl₃): δ -148.6. MS (ESI, *m*/*z,* %): 248.8 ([M + H]⁺, 100), 228.8 ([M - F]⁺, 63).

### EXAMPLE 19. General procedure for the synthesis of intermediates of formula (R)-(VII) and (S)-(VII) (See Scheme 3).

A solution of VO(acac)₂ (0.010 equiv) in anhydrous CHCl₃ (0.25 mL/mmol **VI)** was added to a solution of the chiral ligand 2-[(*E*)-{[(3*R*)-2,2-dimethylpentan-3-yl]imino}methyl]-4,6-diiodophenol (CAS registry number: 850449-43-3) (0.015 equiv) in anhydrous CHCl₃ (0.25 mL/mmol **VI)** under an argon atmosphere and the mixture was stirred at rt for 2 h. Next, a solution of the corresponding intermediate of formula **(VI)** (1.00 equiv) in anhydrous CHCl₃ (0.50 mL/mmol **VI)** was added and the reaction was stirred at rt for 30 min before cooling it to 0 °C (ice bath). After 30 min, hydrogen peroxide (30%, 1.20 equiv) was added and the mixture was stirred vigorously at 0 °C for 20 h. Then the reaction was quenched with 10% aqueous solution of Na₂S₂O₃ (3.30 mL/mmol **VI)** and extracted with DCM (x3). The combined organic layers were dried over Na₂SO₄, filtered and the solvent was evaporated under reduced pressure. The crude residue was purified by column chromatography to afford intermediate of formula **(*R*)-(VII).**

Following the previous general procedure, intermediate of formula (S)-**(VII)** was obtained starting from the corresponding intermediate of formula **(VI),** using the chiral ligand 2-[(E)-{[(3S)-2,2-dimethylpentan-3-yl]imino}methyl]-4,6-diiodophenol (CAS registry number: 916658-65-6).

(*R*)-2-(Fluoromethoxy)-4'-(methylsulfinyl)biphenyl ((*R*)**-VIIa).** Following the previous general procedure, intermediate **(*R*)-VIIa** was obtained starting from **Via** (534 mg, 2.15 mmol) as an oil (335 mg, 59%, >99% ee). Chromatography: hexane to hexane/EtOAc 3:7. 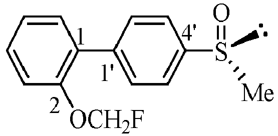 R_{f}: 0.44 (DCM/EtOAc 1:1). [α]_{D}²⁰ = 81.2 (c = 0.24, acetone). IR (ATR): ν 1215 (C-O-C), 1083 (SO), 1046, 960 (C-F). ¹H-NMR (acetone-*d6*): δ 2.75 (s, 3H, CH₃), 5.83 (d, *J* = 54.4, 2H, CH₂F), 7.24 (td, *J* = 7.5, 1.1, 1H, H₅), 7.34 (d, *J =* 7.5, 1H, H₃), 7.44 (d, *J* = 7.4, 1H, H₆), 7.46 (td, *J* = 7.4, 1.7, 1H, H₄), 7.70-7.77 (m, 4H, H2', H₃' , H_{5'}, H_{6'}). ¹³C-NMR (acetone-*d6*): δ 44.2 (CH₃), 101.8 (d, *J =* 216.8, CH₂F), 116.4 (d, *J* = 1.2, C₃), 124.1 (C_{3'}, C_{5'}), 124.6 (C₅), 130.4 (C₄), 131.1 (C_{2'}, C_{6'}), 131.5 (C₁), 131.9 (C₆), 141.2 (C_{1'}), 146.8 (C_{4'}), 154.5 (d, *J =* 3.3 Hz, C₂). ¹⁹F-NMR (acetone-*d6*): δ -150.8. MS (ESI, *m*/*z,* %): 264.8 ([M + H]⁺, 100).

(*S*)-2-(Fluoromethoxy)-4'-(methylsulfinyl)biphenyl **(**(*S*)**-VIIa).** Following the previous general procedure, intermediate **(*S*)-VIIa** was obtained starting from **VIa** (444 mg, 1.78 mmol) as an oil (300 mg, 64%, >99% ee). Chromatography: hexane to hexane/EtOAc 3:7. 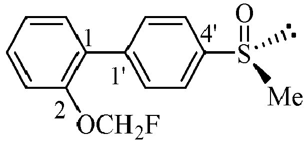 [α]_{D}²⁰ = -80.3 (c = 0.24, acetone). Spectroscopic data were in agreement with those described for enantiomer **(*R*)-VIIa.**

### EXAMPLE 20. General procedure for the synthesis of compounds of formula (R)-(I) and (S)-(I). Method C (See Scheme 3).

Appropriate intermediate of formula **(*R*)-(VII)** (1.00 equiv), ammonium carbamate (4.00 equiv) and (diacetoxyiodo)benzene (3 equiv) were dissolved in methanol (2.0 mL/mmol) and the mixture was stirred at rt for 60 min in an open flask. After completion, the solvent was evaporated under reduced pressure and the residue was purified by column chromatography to afford the desired compound of formula (*R*)-**(I)**.

Following the previous general procedure, compound of formula (S)-**(I)** was obtained starting from the corresponding intermediate of formula (S)-**(VII).**

(*R*)-2-(Fluoromethoxy)-4'-(S-methylsulfonimidoyl)biphenyl **((*R*)-Ik).** Following the previous general procedure, compound was obtained starting from intermediate **(*R*)-VIIa** (335 mg, 1.27 mmol) as a white solid (216 mg, 61%, >99% ee). Chromatography: hexane to hexane/EtOAc 7:3. 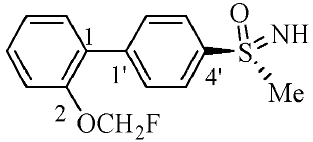 M.p.: 95-96 °C. [α]_{D}²⁰ = -21.5 (c = 0.20, acetone). Spectroscopic data were in agreement with those described for racemic compound **Ik.**

(*S*)-2-(Fluoromethoxy)-4'-(S-methylsulfonimidoyl)biphenyl **((*S*)-Ik).** Following the previous general procedure, compound (*S*)**-Ik** was obtained starting from **(*S*)-VIIa** (282 mg, 1.07 mmol) as a white solid (200 mg, 67%, >99% ee). Chromatography: hexane to hexane/EtOAc 7:3. 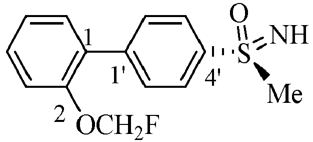 [α]_{D}²⁰ = 20.8 (c = 0.20, acetone). Spectroscopic data were in agreement with those described for racemic compound **Ik.**

### Example 21: Allosteric modulation of human dopamine D1 receptors.

Functional studies at human D1 receptors were carried out in endogenously expressed human D1 receptors in SK-N-MC cell line (ATCC). SK-N-MC cells were thawed and seeded into a black 96-well plate (1×10⁴ cells/well) in Optimem containing 500 µM IBMX.

Cells were treated with the test compounds for 15 min at 25°C and dopamine was added to the corresponding wells and incubated for additional 15 min at 25°C. Treated cells were lysed and the cAMP concentration was measured by homogeneous time-resolved fluorescence energy transfer (HTRF; Cisbio Kit). HTRF was read in a Tecan M1000 Genius Pro reader (λ_{excitation} = 320 nm, λₑₘᵢₛₛᵢₒₙ = 620 nm and 665 nm, 30 flashes).

Data were normalized to dopamine maximum effect and fitted to a 4-parameter logistic equation Prism v2.1 (GraphPad Inc).

The compounds that potentiate more than 25% the dopamine Emax at a fixed concentration of 10 µM were also assayed at different concentrations. If the observed effect is dose-dependent, compounds were then tested in the cAMP assay, this time maintaining a fixed concentration of dopamine (=EC70) while increasing the concentration of the compound, and the potentiation of the dopamine effect was measured.

Compound **Ik** is the most efficient positive allosteric modulator (PAM) of the D1 receptor, showing a high potentiation of dopamine Emax (55% at 10 µM, Table 1) and a high potency in the presence of dopamine (EC₅₀ = 60 nM).

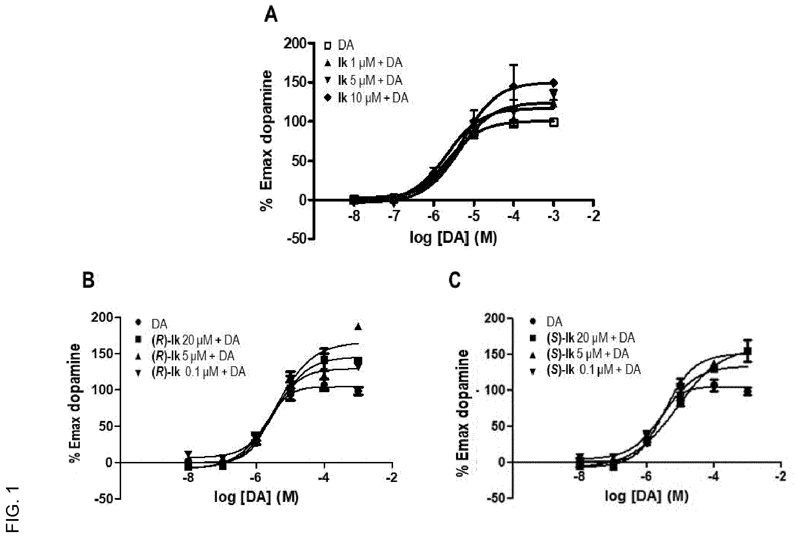

A dose-dependent potentiation of dopamine Emax was observed for **Ik** and its enantiomers **(*R*)-Ik** and **(*S*)-Ik,** with higher enhancements than the racemic compound (63% and 47% vs 35%, at 5 µM) (Table 1 and Figure 1).

**Table 1. Effect of novel compounds of formula (I) in dopamine-induced cAMP production in human neuroblastoma cell line.**

| **Compd** | **Potentiation (%) at 10 µM** |
|---|---|
| **Ia** | 27 |
| **Ib** | 64 |
| **Ic** | 39 |
| **Id** | 25 |
| **Ie** | 33 |
| **If** | 30 |
| **Ig** | 27 |
| **Ih** | 30 |
| **Ii** | 54 |
| **Ij** | 35 |
| **Ik** | 55 (35*) |
| **(*R*)-Ik** | 63* |
| **(*S*)-Ik** | 47* |

| | |
|---|---|
| * values measured at 5 µM. | |

### Example 22. Agonist effect at human dopamine D1 receptors.

SK-N-MC cells seeded into a black 96-well plate (1×10⁴ cells/well) in Optimem containing 500 µM IBMX were treated with the test compounds for 15 min at 25°C. Treated cells were lysed and the cAMP concentration was measured by homogeneous time-resolved fluorescence energy transfer (HTRF; Cisbio Kit). HTRF was read in a Tecan M1000 Genius Pro reader (λ_{excitation} *=* 320 nm, λₑₘᵢₛₛᵢₒₙ = 620 nm and 665 nm, 30 flashes).

Data were normalized to dopamine maximum effect and fitted to a 4-parameter logistic equation Prism v2.1 (GraphPad Inc).

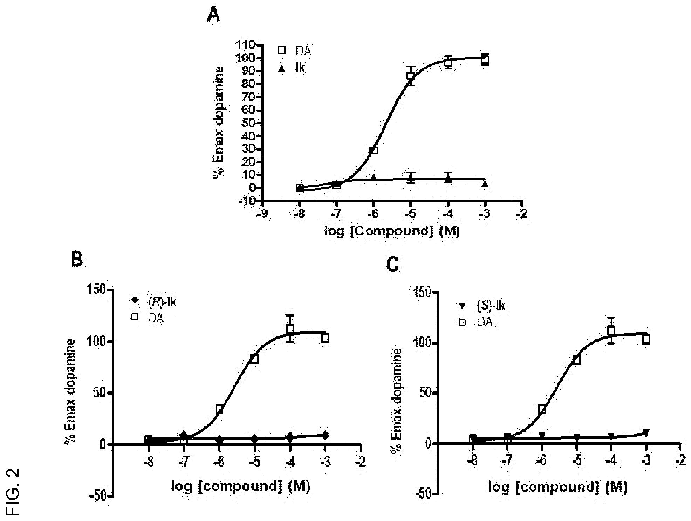

Importantly, no potentiation was observed for compounds **Ik,** (*R*)-**Ik** and **(*S*)-Ik** in the absence of dopamine, so they do not display intrinsic agonist activity (Figure 2).

### Example 23. Functional assays at human D2 receptors.

Functional studies at D2 receptors were carried out in recombinant human D2 receptors stably transfected in-house in a CHO cell line. Cells were thawed and seeded into a 96-well plate (5×10³ cells/well) in Optimem containing 500 µM IBMX.

Cells were treated with the test compounds for 5 min at 37°C and dopamine was added to the corresponding wells and incubated for additional 10 min at 37°C. After this time cells were treated with 10 µM forskolin for 5 min at 37°C. Treated cells were lysed and the cAMP concentration was measured by homogeneous time-resolved fluorescence energy transfer (HTRF; Cisbio Kit). HTRF was read in a Tecan M1000 Genius Pro reader (λ_{excitation} = 320 nm, λₑₘᵢₛₛᵢₒₙ = 620 nm and 665 nm, 30 flashes).

Data were normalized to dopamine maximum effect and fitted to a 4-parameter logistic equation Prism v2.1 (GraphPad Inc).

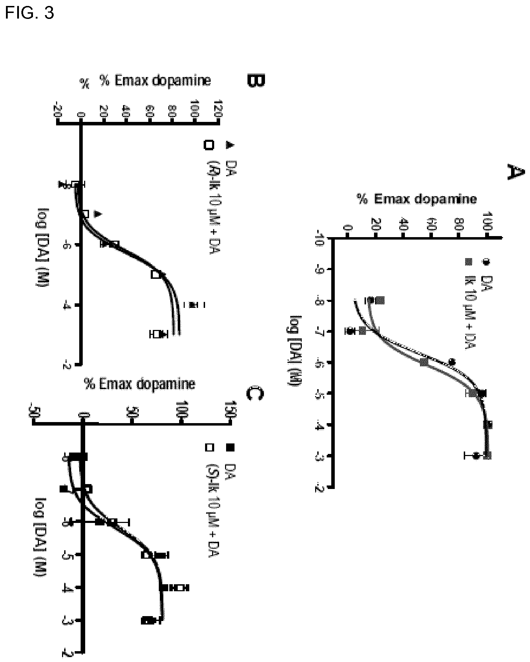

Notably, compounds **Ik,** (*R*)**-Ik** and (*S*)**-Ik** exhibited functional subtype selectivity against dopamine D2 receptor (Figure 3).

### Example 24. Functional assays at human D3 and D4 receptors.

Functional studies at both human D3 and D4 receptors receptors were carried by employing a PatHunter Beta-arrestin express GPCR assay kit from DiscoverX. Cells were plated maintained for 48 h at 37°C in a 5% CO₂ atmosphere in cell plating reagen t provided in the kit.

Cells were treated with the test compounds for 30 min at 37°C and dopamine was added to the corresponding wells and incubated for additional 90 min at 37°C. Cells were then treated with the working detection solution provided with the kit for 1 h and beta-arrestin translocation was measured by luminescence detection (integration time=500 ms) in a Tecan M1000 Genius Pro reader.

Data were normalized to dopamine maximum effect and fitted to a 4-parameter logistic equation Prism v2.1 (GraphPad Inc).

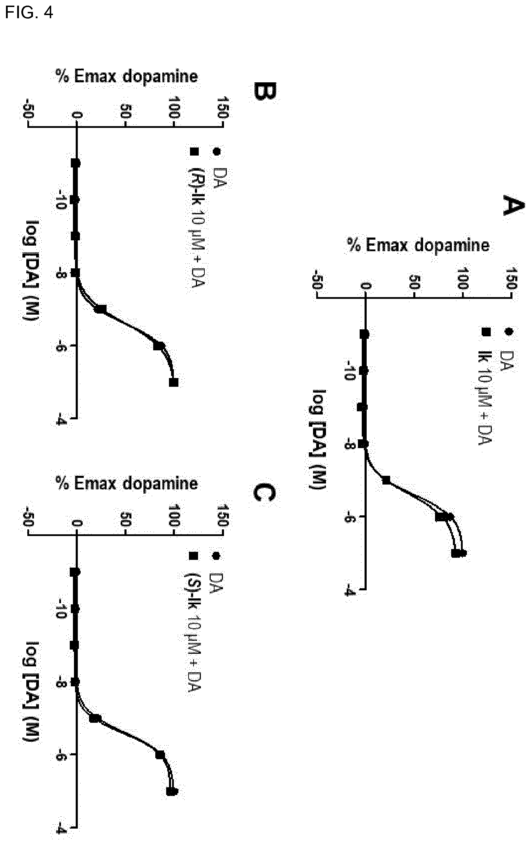

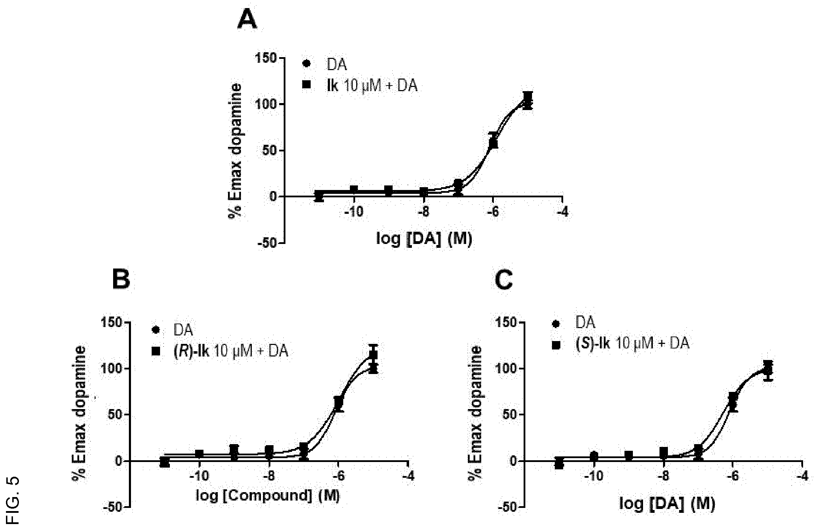

Notably, compounds **Ik,** (*R*)**-Ik** and (*S*)**-Ik** exhibited functional subtype selectivity against D3 and D4 receptors (Figures 4 and 5).

### Example 25. Functional assays at human D5 receptors.

Functional studies at human D5 receptors receptors were carried by employing a cAMP Hunter express DRD5 assay kit from DiscoverX. Cells were plated maintained for 24 h at 37°C in a 5% CO₂ atmosphere in cell plating reagent provided in the kit.

Cells were treated with the test compounds for 15 min at 37°C and dopamine was added to the corresponding wells and incubated for additional 30 min at 37°C. Cells were then treated with the cAMP detection solution provided with the kit for 1 h and then cAMP solution A was added to each well and incubated for 3 h at RT. cAMP formation was measured by luminescence detection (integration time=500 ms) in a Tecan M1000 Genius Pro reader.

Data were normalized to dopamine maximum effect and fitted to a 4-parameter logistic equation Prism v2.1 (GraphPad Inc).

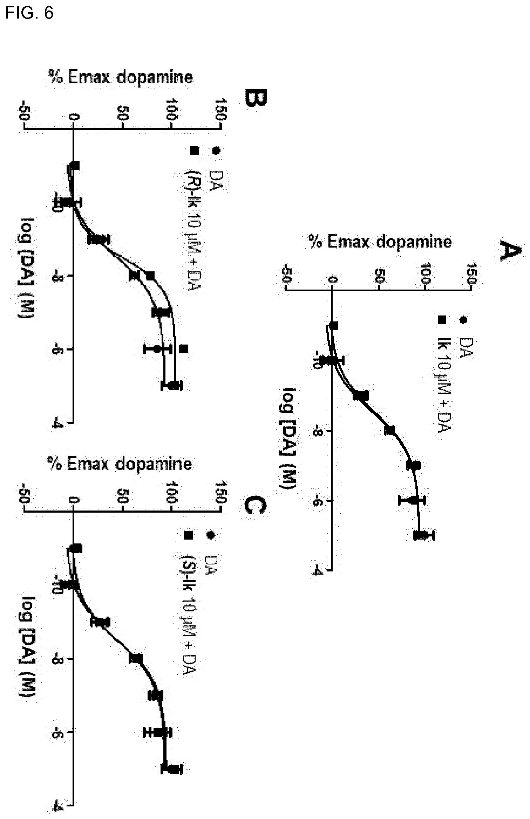

Notably, compounds **Ik,** (*R*)**-Ik** and (*S*)**-Ik** exhibited functional subtype selectivity against D5 receptor (Figure 6).

### Example 26. Radioligand binding assays at human D1 receptors.

Assays were carried out by using plasma membranes (12 µg/well) expressing human dopamine D1 receptor (Perkin Elmer). Membranes were incubated in a Multiscreen FC 96-well plate (Millipore) with 0.7 nM [³H]SCH23390 (Perkin Elmer) and increasing amounts of the test compound in assay buffer (50 mM Tris-HCl, 5 mM MgCl₂; pH=7.4) for 1 h at 27°C. After this time the well content was filtered through a Millipore manifold and membranes washed 4 times with assay buffer. Plate was dried and radioactivity was measured in a Microbeta Trilux liquid scintillation reader (Perkin-Elmer). Non-specific binding was determined in the presence of 1 µM (+)-butaclamol.

Data were normalized to percentage of specific binding and fitted to a 4-parameter logistic equation with Prism v2.1 (GraphPad Inc).

Results revealed no significant binding of compounds **Ik,** (*R*)**-Ik** and (*S*)**-Ik** to the orthosteric site (36%, 12% and 6% of radioligand displacement, respectively, at 10 µM), in contrast to the full displacement by dopamine and haloperidol.

### REFERENCES

- J Pharmacol Exp Ther (2015) 354: 340-349 Lewis MA *et al.*
- WO2014193781A1
- J Pharmacol Exp Ther (2010) 332(3): 876-85 Soriano A *et al.*

## Claims

1. A compound of formula **(I),** a pharmaceutically acceptable salt thereof or any stereoisomer either of the compound of formula **(I)** or of a pharmaceutically acceptable salt thereof: 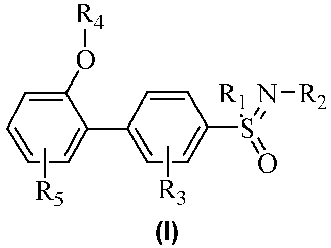 wherein:
R₁ is selected from the group consisting of (C₁-C₄)-alkyl;
R₂ is selected from the group consisting of H and (C₁-C₄)-alkyl;
R₃ is selected from the group consisting of H, (C₁-C₄)-alkyl and halogen;
R₄ is selected from the group consisting of CF₃, CHF₂, CH₂F, (C₁-C₄)-alkylCF₃, (C₁-C₄)-alkylCHF₂ and (C₁-C₄)-alkylCH₂F; and
R₅ is selected from the group consisting of H, halogen, hydroxyl, (C₁-C₄)-alkyl, methoxy and O(C₁-C₄)-alkyl.

2. The compound according to claim 1, wherein
R₁ is selected from -CH₃, -CH₂CH₃, -CH₂CH₂CH₃ and -CHCH₃CH₃;
R₂ is selected from H, -CH₃, -CH₂CH₃, -CH₂CH₂CH₃ and -CHCH₃CH₃; and
R₄ is selected from CF₃, CHF₂, CH₂F, -CH₂CF₃, -CH₂CHF₂, -CH₂CH₂F, -CH₂CH₂CF₃, -CH₂CH₂CHF₂ and -CH₂CH₂CH₂F.

3. The compound according to any one of claims 1-2, wherein
R₃ is selected from H, -CH₃, -CH₂CH₃, F, Cl and Br; and
R₅ is selected from H, F, Cl, Br, -OH, -CH₃ and -CH₂CH₃.

4. The compound according to any one of claims 1-3, wherein
R₁ is selected from -CH₃, -CH₂CH₃, -CH₂CH₂CH₃ and -CHCH₃CH₃;
R₂ is selected from H, -CH₃, -CH₂CH₃, -CH₂CH₂CH₃ and -CHCH₃CH₃;
R₃ is selected from H, -CH₃, -CH₂CH₃, F, Cl and Br;
R₄ is selected from CF₃, CHF₂, CH₂F, -CH₂CF₃, -CH₂CHF₂, -CH₂CH₂F, -CH₂CH₂CF₃, -CH₂CH₂CHF₂ and -CH₂CH₂CH₂F; and
R₅ is selected from H, F, Cl, Br, -OH, -CH₃ and -CH₂CH₃.

5. The compound according to any one of claims 1-4, wherein
R₁ is selected from -CH₃ and -CH₂CH₃;
R₂ is selected from H and -CH₃;
R₃ is selected from H and Cl;
R₄ is selected from CF₃, CHF₂ and CH₂F; and
R₅ is H.

6. The compound according to any one of claims 1-5, selected from 2-(difluoromethoxy)-4'-(*S*-methylsulfonimidoyl)biphenyl **(Ia);** 2-(difluoromethoxy)-4'-(*N*,*S*-dimethylsulfonimidoyl)biphenyl **(Ib);** 2-(difluoromethoxy)-4'-(*S*-ethylsulfonimidoyl)biphenyl (**Ic**)**;** 3'-chloro-2-(difluoromethoxy)-4'-(*S*-methylsulfonimidoyl)biphenyl **(Id);** 3'-chloro-2-(difluoromethoxy)-4'-(*N*,*S*-dimethylsulfonimidoyl)biphenyl (**Ie**)**;** 3'-chloro-4'-(*S*-methylsulfonimidoyl)-2-(trifluoromethoxy)biphenyl **(If);** 3'-chloro-2-(trifluoromethoxy)-4'-(*N*,*S*-dimethylsulfonimidoyl)biphenyl **(Ig);** 4'-(*N*,*S*-dimethylsulfonimidoyl)-2-(fluoromethoxy)biphenyl **(Ih);** 2-(fluoromethoxy)-4'-(*S*-ethylsulfonimidoyl)biphenyl **(Ii);** 3'-chloro-2-(fluoromethoxy)-4'-(*N*,*S*-dimethylsulfonimidoyl)biphenyl **(Ij);** 2-(fluoromethoxy)-4'-(*S*-methylsulfonimidoyl)biphenyl **(Ik);** (R)-2-(fluoromethoxy)-4'-(*S*-methylsulfonimidoyl)biphenyl **((R)-Ik);** (*S*)-2-(fluoromethoxy)-4'-(*S*-methylsulfonimidoyl)biphenyl **((*S*)-Ik).**

7. A compound of formula **(I),** a pharmaceutically acceptable salt thereof or any stereoisomer either of the compound of formula **(I)** or of a pharmaceutically acceptable salt thereof, as defined in any one of claims 1-6, for use as a medicament.

8. A compound according to any one of claims 1-6 for use in a method of treating disorders associated with dopamine D1 receptors.

9. A compound according to any one of claims 1-6 for use in the treatment of a disorder selected from schizophrenia, bipolar disorders, autism spectrum disorder, Parkinson's disease, schizophrenia, Alzheimer's disease, senile dementia, HIV-associated dementia, Lewy body dementia, vascular dementia, frontotemporal dementia, Huntington's chorea, Tourette's syndrome, restless leg syndrome (RLS), or tardive dyskinesia, anxiety, depression, major depressive disorder (MDD), treatment-resistant depression (TRD), bipolar disorder, chronic apathy, anhedonia, chronic fatigue, post-traumatic stress disorder, seasonal affective disorder, social anxiety disorder, post-partum depression, serotonin syndrome, attention deficit hyperactivity disorder (ADHD), drowsiness, excessive daytime sleepiness, inattention, impulsivity, compulsive gambling, mild substance abuse, drug dependence and drug abuse relapse; sexual dysfunction, migraine, post-ischemic tubular necrosis, renal failure, hyponatremia, resistant edema, hypertension, congestive heart failure, cardiogenic or septic shock or postoperative ocular hypotonia, obesity, diabetes, hyperglycemia, atherosclerosis and dyslipidemia.

10. The compound according to any one of claims 1-6 for use in the treatment of cognitive impairment and negative symptoms in schizophrenia, bipolar disorders and autism spectrum disorder; mild cognitive impairment associated to Parkinson's disease, schizophrenia, Alzheimer's disease, senile dementia, HIV-associated dementia, Lewy body dementia, vascular dementia, or frontotemporal dementia; motor symptoms in Parkinson's disease.

11. The compound according to any one of claims 1-6 for use in the treatment of cognitive impairment and negative symptoms in schizophrenia and mild cognitive impairment associated to Parkinson's disease.

12. A compound according to any one of claims 1-6 for use in the treatment of Parkinson's disease or schizophrenia.

13. A pharmaceutical composition comprising a therapeutically effective amount of a compound of formula **(I),** a pharmaceutically acceptable salt thereof or any stereoisomer either of the compound of formula **(I)** or of a pharmaceutically acceptable salt thereof as defined in any of the claims 1-6, together with one or more pharmaceutically acceptable diluents, excipients or carriers.

14. A process for the preparation of a compound of formula **(I),** a pharmaceutically acceptable salt thereof or any stereoisomer either of the compound of formula **(I)** or of a pharmaceutically acceptable salt thereof, as defined in any one of claims 1-6, comprising:
a) oxidation of a compound of formula **(VI)** to obtain the sulfoxide intermediate of formula **(VII)** 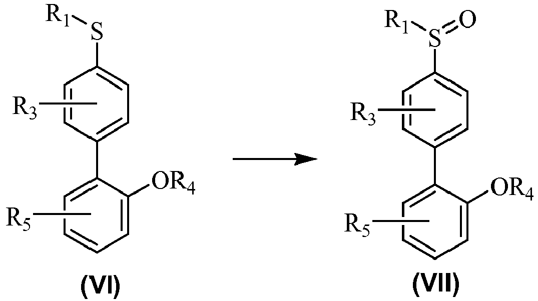 wherein R₁, R₃, R₄ and R₅ are as defined above; and
b) imination of intermediate of formula **(VII),** optionally followed by *N-*alkylation, to obtain compounds of formula **(I).**

15. The process according to claim 14, wherein in step a) an asymmetric oxidation of compound of formula **(VI)** is performed to obtain the corresponding enantiomerically pure sulfoxide intermediate of formula (R)-**(VII)** or **(S)-(VII).**
